# FASCICULE DE BREVET EUROPEEN

(11) **EP 2 252 258 B1**
(45) Date de publication et mention de la délivrance du brevet: **19.09.2018**
(21) Numéro de dépôt: 09710793.2
(22) Date de dépôt: 11.02.2009
(51) Int. Cl.: A61K 8/99, A61K 8/02, A61Q 19/00, A61Q 19/08, A61Q 5/00, A61Q 5/12, A61Q 7/00

(54) **UTILISATION DE SUBSTANCES ACTIVES NATURELLES DANS DES COMPOSITIONS COSMÉTIQUES OU THÉRAPEUTIQUES**
VERWENDUNG VON NATUERLICHEN AKTIVEN INHALTSTOFFEN IN KOSMETISCHEN UND THERAPEUTISCHEN ZUSAMMENSETZUNGEN
USE OF NATURAL ACTIVE SUBSTANCES IN COSMETIC OR THERAPEUTIC COMPOSITIONS

(30) Priorité: 12.02.2008 FR 0800754
(43) Date de publication de la demande: 24.11.2010
(73) Titulaire: Lesaffre et Compagnie, 75001 Paris (FR)
(72) Inventeur: JUSTEN, Peter, F-92500 Rueil-Malmaison (FR); BORREILL, Dominique, Marie, Noëlle, F-94430 Chennevières-sur-Marne (FR); MARQUES, William, F-92500 Rueil-Malmaison (FR)
(74) Mandataire: Cabinet Plasseraud
(86) Numéro de dépôt international: PCT/IB2009/000237
(87) Numéro de publication internationale: WO 2009/101503

(56) Documents cités:
- EP-A- 0 237 398
- WO-A-02/03943
- WO-A-96/29048
- WO-A-03/008458
- WO-A-03/068824
- WO-A-2008/015343
- JP-A- 2003 238 384
- US-A1- 2006 165 644

## Description

### DOMAINE DE L'INVENTION

La présente invention a pour objet l'utilisation de substances actives naturelles dans des compositions cosmétiques, ainsi que dans des compositions thérapeutiques, ces substances étant des protéines de levure hydrolysées issues de la fraction insoluble des levures.

### ARRIERE-PLAN TECHNOLOGIQUE

Les hydrolysats de protéines ont suscité depuis plusieurs années un intérêt pour des applications cosmétiques ou thérapeutiques.

Les hydrolysats de protéines peuvent avoir différentes origines : animale, en particulier de poisson, végétale, ou fongique, par exemple la levure.

La présence ou l'absence d'activité biologique d'un hydrolysat de protéines dépend notamment de la nature des protéines de départ.

Ainsi, l'hydrolyse de protéines de poisson a permis d'obtenir des protéines hydrolysées possédant une structure spatiale particulière reconnue par des récepteurs. Des activités de type hormonal et opioïde ont ainsi été mises en évidence (Legal et Stenberg, Biofutur, N°179, 1998, pages 61-63).

Certains documents de l'art antérieur mentionnent l'utilisation d'hydrolysats de protéines de levure dans des compositions cosmétiques. Ces hydrolysats de protéines de levure sont obtenus soit par hydrolyse de cellules de levure entières, soit par hydrolyse de la fraction soluble de la levure (le contenu cytoplasmique).

Ainsi, la demande de brevet EP 0 695 801 décrit l'utilisation cosmétique d'une composition peptidique obtenue par :
- une étape de traitement thermique de levures suivi d'un traitement avec des enzymes lytiques de la paroi cellulaire des levures, pour obtenir un mélange,
- une étape de purification et séparation des protéines dudit mélange, pour obtenir des protéines de levure, et
- une étape d'hydrolyse desdites protéines.

Les enzymes lytiques de la paroi cellulaire utilisées dans ce document sont des enzymes qui attaquent les glucanes et déstabilisent la paroi et la membrane. Une telle composition comprend donc des protéines hydrolysées issues de la fraction soluble de la levure.

La demande de brevet EP 0 126 364 décrit l'utilisation cosmétique d'un produit sans histamine, apyrogène, stérile, actif, qui est obtenu par un procédé comprenant les étapes suivantes :
- plasmolyse de levures et homogénéisation à une température inférieure à 0°C,
- traitement avec une enzyme protéolytique, pendant au moins 70h,
- traitement avec une diamine oxydase afin d'éliminer les substances contenant de l'histamine,
- précipitation fractionnée avec un mélange d'alcools pour éliminer les protéines résiduelles.

Le produit final comprend donc des protéines de levure hydrolysées issues de l'hydrolyse des protéines de la levure entière. La mise en oeuvre d'un tel procédé de production présente plusieurs inconvénients, dont notamment la longueur du procédé, la multiplicité des étapes et la nécessité de travailler en conditions stériles.

La demande de brevet EP 0 237 398 décrit l'utilisation cosmétique de polypeptides biologiquement actifs obtenus par le procédé comprenant les étapes de :
- broyage mécanique de substances naturelles, par exemple des levures, pour obtenir un homogénéisat aqueux,
- hydrolyse enzymatique avec un agent d'hydrolyse comportant l'a-chymotrypsine et éventuellement de la trypsine, pour obtenir un hydrolysat,
- séparation d'une fraction de polypeptides d'un poids moléculaire déterminé.

La fraction polypeptidique provient donc de l'hydrolyse de protéines issues de levures entières. La fraction de polypeptides a notamment un poids moléculaire inférieur à 10 000 Da et supérieur à 1000 Da.

Les consommateurs sont de plus en plus demandeurs de produits « naturels », que ce soit dans le domaine alimentaire, cosmétique ou pharmaceutique.

Dans le domaine cosmétique et pharmaceutique, il y a un réel besoin de fournir de nouvelles substances actives naturelles qui :
- présentent des qualités cosmétiques ou thérapeutiques améliorées, telles que des effets hydratant, anti-vieillissement et /ou fermeté ; et/ou
- une excellente stabilité au cours du temps ; et/ou
- dont la production est homogène et/ou dont le procédé de production est facilement mis en oeuvre à l'échelle industrielle.

### RESUME DE L'INVENTION

La présente invention a pour objet de fournir des substances actives naturelles utiles dans le domaine cosmétique ou thérapeutique.

Un objet de l'invention est également de fournir de nouvelles compositions cosmétiques ou thérapeutiques.

Un autre objet de l'invention concerne une méthode de traitement cosmétique de la peau et/ou des phanères et/ou des muqueuses ou ces substances pour leur utilisation thérapeutique.

La présente invention repose notamment sur la découverte d'une nouvelle catégorie de protéines hydrolysées possédant des activités cosmétiques et/ou thérapeutiques améliorées, et/ou une excellente stabilité au cours du temps, et/ou dont la production est homogène et/ou le procédé de production facilement mis en oeuvre à l'échelle industrielle.

La présente invention a pour objet une composition cosmétique ou thérapeutique selon la présente revendication 1, comprenant des protéines de levure hydrolysées à titre de substance active, caractérisée en ce que lesdites protéines de levure hydrolysées sont issues de la fraction insoluble des levures.

Selon un mode de réalisation, les protéines de levure hydrolysées sont obtenues par hydrolyse enzymatique et/ou hydrolyse acide et/ou hydrolyse alcaline.

Selon un mode de réalisation, les protéines de levure hydrolysées sont obtenues par hydrolyse enzymatique avec au moins une peptidase, de préférence choisie parmi la papaïne, trypsine, chymotrypsine, subtilisine, pepsine, thermolysine, pronase, flavastacine, enterokinase, protéase facteur Xa, furine, bromélaïne, protéinase K, genenase I, thermitase, carboxypeptidase A, carboxypeptidase B, collagénase, et/ou leur mélange.

Selon un mode de réalisation, les protéines de levure hydrolysées sont issues de levures du genre *Saccharomyces*, *Kluyveromyces*, *Torula*, *Candida, Hansenula, Pichia,* et/ou leur mélange, de préférence *Saccharomyces,* avantageusement *Saccharomyces cerevisiae.*

Selon un mode de réalisation, les protéines de levures hydrolysées comportent au moins 40%, de préférence au moins 45%, plus préférentiellement au moins 50%, encore plus préférentiellement au moins 55%, encore plus préférentiellement au moins 60% de protéines de levure avec un poids moléculaire compris de 1 à 5 kDa.

Selon un mode de réalisation, les protéines de levures hydrolysées comportent au plus 55%, de préférence au plus 50%, plus préférentiellement au plus 45%, encore plus préférentiellement au plus 40%, encore plus préférentiellement au plus 35% de protéines de levure hydrolysées avec un poids moléculaire inférieur à 1 kDa.

Selon un mode de réalisation, le rapport AN/TN des protéines de levure hydrolysées est inférieur ou égal à 35%, notamment inférieur ou égal à 30%, notamment inférieur ou égal à 25%, notamment inférieur ou égal à 20%.

Selon un mode de réalisation, la composition comprend 0,001% à 20% de protéines de levure hydrolysées, plus préférentiellement de 0,001 % à 15 % de protéines de levure hydrolysées, encore plus préférentiellement de 0,001% à 10% de protéines de levure hydrolysées, encore plus préférentiellement de 0,01% à 3% de protéines de levure hydrolysées, encore plus préférentiellement de 0,01% à 2% de protéines de levure hydrolysées.

Selon un mode de réalisation, la composition comprend au moins un additif choisi parmi les conservateur, chélateur, colorant, filtre UV, régulateur de pH, texturant, parfum ou antioxydant, et/ou au moins un excipient choisi parmi des composés hydrophiles, des composés hydrophobes ou des tensioactifs.

La présente invention a également pour objet un procédé de préparation d'une composition cosmétique ou thérapeutique, comprenant les étapes de :
- hydrolyse de protéines de la fraction insoluble des levures, pour obtenir des protéines de levure hydrolysées, et
- mélange desdites protéines de levure hydrolysées avec un véhicule cosmétique ou thérapeutique acceptable.

La présente invention a pour objet l'utilisation de protéines de levure hydrolysées issues de la fraction insoluble des levures à titre de substance active dans des compositions cosmétiques et / ou thérapeutiques.

Un autre objet de l'invention concerne une méthode de traitement cosmétique comprenant une étape de mise en contact sur la peau et/ou les phanères et/ou les muqueuses d'une composition selon l'invention ou telle que susceptible d'être obtenue par le procédé selon l'invention.

Un autre objet de l'invention concerne également des protéines de levure hydrolysées issues de la fraction insoluble des levures pour leur utilisation en tant que médicament, de préférence pour le traitement et / ou la prévention de la sécheresse cutanée pathologique, des problèmes de cicatrisation pathologique et/ou l'hyperséborrhée pathologique, et/ou l'acné.

### BREVE DESCRIPTION DES FIGURES

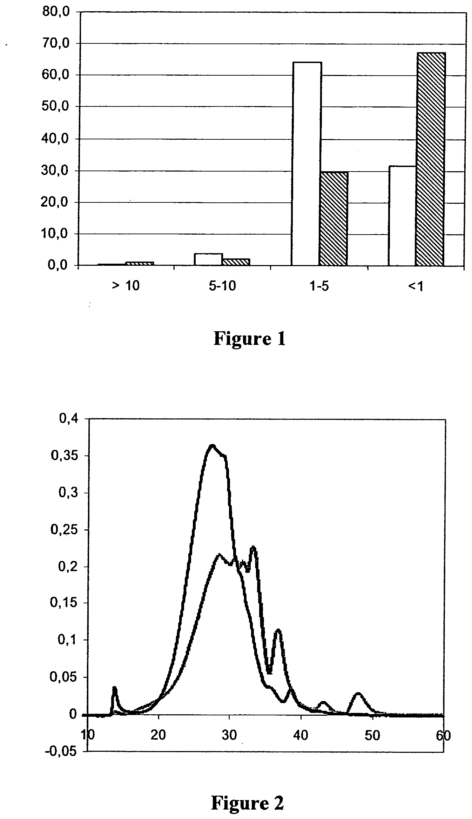

La figure 1 représente, en pourcentage, la répartition de taille (en kDa) au sein des protéines de levure hydrolysées selon l'invention (histogramme blanc) et au sein de protéines de levure hydrolysées issues de l'hydrolyse de cellules entières de levure (histogramme rayé).
La figure 2 représente le profil de poids moléculaire des protéines de levure hydrolysées selon l'invention (courbe noire) et celui de protéines de levure hydrolysées issues de l'hydrolyse de cellules entières de levure (courbe grise). L'axe des ordonnées indique l'absorbance lue à 214 nm et l'axe des abscisses le temps de rétention en minutes.

### DESCRIPTION DETAILLEE DE MODES DE REALISATION

La présente invention a pour objet une composition cosmétique ou thérapeutique selon la présente revendication 1, comprenant des protéines de levure hydrolysées à titre de substance active.

La présente invention a notamment pour objet une composition cosmétique ou thérapeutique comprenant des protéines de levure hydrolysées à titre de substance active, caractérisée en ce que lesdites protéines de levure hydrolysées sont issues de la fraction insoluble des levures.

Les protéines de levure hydrolysées sont également appelées « peptones de levure » ou « peptides de levure obtenus par hydrolyse».

Par « composition cosmétique », on désigne ici une composition destinée à procurer un effet cosmétique.

Dans un mode de réalisation préféré selon l'invention, l'effet cosmétique est obtenu par une application topique des compositions selon l'invention.

Le terme « topique » indique que la composition est active à l'endroit où elle est appliquée, sur la peau, les phanères et/ou les muqueuses. La composition selon l'invention peut à la fois cibler les couches superficielles de l'épiderme, et / ou le derme.

Par le terme « phanère », on désigne de manière générale tout ce qui recouvre la peau, et notamment les cheveux, ongles, poils, cils.

Le terme « peau » englobe le cuir chevelu.

Le terme « peau » englobe le derme et l'épiderme, dont les couches superficielles de l'épiderme.

Par le terme « muqueuse » ou « tissu épithélial humide », on désigne les membranes qui tapissent les cavités ouvertes vers le milieu extérieur, et en particulier les muqueuses buccales, nasales et génitales, dont les muqueuses vaginales.

Dans un autre mode de réalisation préféré, l'effet cosmétique est obtenu par une administration par voie orale.

Par « composition thérapeutique», on désigne une composition destinée à procurer un effet thérapeutique.

Une composition thérapeutique préférée selon l'invention est une composition dermatologique.

L'effet thérapeutique est en particulier obtenu par une application topique des compositions thérapeutiques selon l'invention.

La présente invention a donc également pour objet une composition cosmétique ou thérapeutique telle que définie ci-dessus destinée à une application sur la peau et/ou sur les phanères et/ou les muqueuses.

Une autre composition préférée selon l'invention est une composition appropriée pour une administration par voie orale.

Par « substance active » ou « principe actif » ou « matière active », on désigne ici la substance responsable de l'effet cosmétique dans le cas d'une composition cosmétique ou responsable de l'effet thérapeutique dans le cadre d'une composition thérapeutique.

Une composition cosmétique selon l'invention comprend au moins un composé à titre de substance active et un véhicule cosmétique acceptable.

Une composition thérapeutique selon l'invention comprend au moins un composé à titre de substance active et un véhicule thérapeutique acceptable.

Les protéines de levure hydrolysées selon l'invention sont issues de la fraction insoluble des levures.

Par « fraction insoluble », on désigne les écorces de levure, c'est-à-dire à la fois la paroi et la membrane plasmique des levures.

La fraction insoluble représente environ 20 à 30% en masse des matières sèches des cellules de levure.

Par « fraction soluble », on désigne le contenu de la levure autre que les écorces de levure.

Les écorces de levure comprennent essentiellement des glucides (environ 50%). Les matières protéiques représentent environ 10% à 20% des écorces de levure, notamment environ 13 à 18% des écorces de levure (en masse de matières sèches).

La fraction insoluble peut être obtenue par un traitement thermique de la levure pendant 1 à 3 h entre 70°C et 90°C, suivi d'une séparation de la fraction soluble et insoluble, notamment par centrifugation. La fraction soluble est alors éliminée et la fraction insoluble est récupérée.

Les protéines de levures hydrolysées selon l'invention sont obtenues par hydrolyse des protéines issues de la fraction insoluble des levures.

Les protéines de levure hydrolysées peuvent être soumises à des traitements complémentaires spécifiques (par exemple, séparation des protéines par centrifugation, concentration, filtration ou traitement au charbon actif).

Ainsi, contrairement aux hydrolysats de protéines classiques, obtenus après une autolyse ou une hydrolyse enzymatique de l'ensemble du contenu cellulaire ou seulement de la partie soluble, les protéines de levure hydrolysées selon l'invention sont issues d'une fraction cellulaire particulière. Les protéines de la fraction insoluble de la levure sont en effet de nature différente de celles figurant dans la fraction soluble de la levure. Les protéines de la fraction insoluble comprennent notamment des mannoprotéines qui sont absentes de la fraction cellulaire.

De plus, les protéines de la fraction insoluble sont essentiellement des protéines natives, n'ayant subi aucune hydrolyse, alors que les protéines de la fraction soluble ont pour la plupart déjà subi des hydrolyses partielles ou totales. Ainsi, le résultat de l'hydrolyse effectuée à partir de protéines issues de la levure entière est moins contrôlable du fait de l'hétérogénéité de l'état des protéines de départ.

De plus, le procédé selon l'invention permet de rendre plus accessibles les protéines de la fraction insoluble à l'hydrolyse, entre autres par une forte concentration desdites protéines, par rapport à une hydrolyse effectuée sur une levure entière.

Ainsi, les protéines de levure hydrolysées selon l'invention sont caractérisées par une nature des peptides particulière, un profil de répartition moléculaire des peptides particulier (et homogène), et/ou un ratio AN/TN spécifique (avec très peu d'acides aminés libres), savoir notamment un rapport AN/TN des protéines de levure hydrolysées est inférieur ou égal à 35%, notamment inférieur ou égal à 30%, notamment inférieur ou égal à 25%, notamment inférieur ou égal à 20%.

Par « rapport AN/TN », on désigne le rapport de la quantité d'azote des acides aminés (en pourcentage) sur la quantité d'azote total (en pourcentage). Le ratio AN/TN indique le taux de dégradation des protéines, en particulier le degré d'hydrolyse des protéines de levure.

De manière surprenante, cette nouvelle source de protéines de levures, une fois hydrolysées, possède des activités cosmétique et thérapeutiques.

Les protéines de levure hydrolysées se présentent sous une forme sèche, notamment sous forme de poudre, ou en solution, par exemple en solution aqueuse.

La présente invention a pour objet une composition cosmétique ou thérapeutique telle que définie ci-dessus, caractérisée en ce que les protéines de levure hydrolysées sont obtenues par hydrolyse enzymatique et/ou hydrolyse acide et/ou hydrolyse alcaline.

L'hydrolyse acide est une hydrolyse obtenue en milieu acide, de préférence à chaud, par exemple en utilisant un acide fort comme l'acide chlorhydrique, acide sulfurique, acide phosphorique et / ou acide nitrique.

L'hydrolyse acide détruit notamment le tryptophane et transforme les acides aminés glutamine et asparagine respectivement en glutamate et aspartate.

L'hydrolyse alcaline est une hydrolyse obtenue en milieu alcalin, par exemple en utilisant une base forte comme l'hydroxyde de sodium ou l'hydroxyde de potassium.

L'hydrolyse alcaline détruit notamment les acides aminés sérine, thréonine, cystéine.

L'hydrolyse enzymatique des protéines de levure est réalisée au moyen d'hydrolases.

Selon un mode de réalisation préféré, les protéines de levure hydrolysées selon l'invention sont obtenues par hydrolyse enzymatique.

L'hydrolyse enzymatique est effectuée par ajout d'au moins une enzyme exogène. De préférence, les enzymes endogènes de la levure ont été préalablement inactivées, par exemple par un traitement thermique.

Les hydrolases selon l'invention sont notamment des hydrolases agissant sur les liaisons peptidiques. De telles hydrolases, appelées peptidases ou protéases ou enzymes protéolytiques, portent le numéro EC 3.4 dans la classification EC. Les peptidases catalysent le clivage hydrolytique de liaison C-N.

Les hydrolases selon l'invention sont notamment choisies parmi les exopeptidases, -en particulier aminopeptidase, dipeptidase, dipeptidyl-peptidase, tripeptidyl-peptidase, peptidyl-dipeptidase, carboxypeptidase de type sérine, carboxypeptidase de type cystéine, métallocarboxypeptidase, peptidase oméga -, et les endopeptidases (ou protéinase), en particulier endopeptidase sérine, endopeptidase cystéine, endopeptidase aspartique, métalloendopeptidase.

L'hydrolyse enzymatique peut être couplée à une hydrolyse des ponts disulfure, réalisée au moyen d'agents réducteurs, par exemple le 2-mercaptoéthanol ou le dithiothréitol, le TCEP (Tris (2-carboxyethyl) phosphine).

La présente invention a particulièrement pour objet une composition cosmétique ou thérapeutique telle que définie ci-dessus, caractérisée en ce que les protéines de levure hydrolysées sont obtenues par hydrolyse enzymatique avec au moins une peptidase, de préférence choisie parmi la papaïne, trypsine, chymotrypsine, subtilisine, pepsine, thermolysine, pronase, flavastacine, enterokinase, protéase facteur Xa, furine, bromélaïne, protéinase K, genenase I, thermitase, carboxypeptidase A, carboxypeptidase B, collagénase, alcalase®, neutrase® et/ou leur mélange.

Les conditions d'utilisation des enzymes (notamment leur concentration, la durée de l'hydrolyse, la température) sont facilement déterminées par l'homme du métier.

A titre d'exemple, l'hydrolyse peut être effectuée par ajout de protéases pendant au moins 18 h entre 45°C et 55°C.

De préférence, la partie solubilisée comprenant les protéines de levure hydrolysées est alors récupérée par centrifugation, avant d'être éventuellement concentrée, puis séchée.

Une enzyme préférée selon l'invention est choisie parmi la papaïne, trypsine, pepsine, alcalase® et/ou neutrase®.

Dans un mode de réalisation particulier, l'hydrolyse enzymatique est obtenue avec au moins deux enzymes, notamment au moins trois enzymes, notamment au moins 4 enzymes différentes.

A titre d'exemple, l'hydrolyse enzymatique peut être réalisée avec un mélange de papaïne et alcalase®.

La présente invention concerne notamment une composition cosmétique ou thérapeutique telle que définie ci-dessus, caractérisée en ce que les protéines de levure hydrolysées sont issues des levures du genre *Saccharomyces, Kluyveromyces, Torula*, *Candida*, *Hanseluna*, *Pichia* et/ou leur mélange, de préférence *Saccharomyces,* avantageusement *Saccharomyces cerevisiae.*

Les protéines de levure hydrolysées issues de levures du genre *Hansenula* sont de préférence des levures *Hansenula anomala.*

Les protéines de levure hydrolysées issues de levures du genre *Pichia* sont de préférence des levures *Pichia pastoris.*

Les protéines de levure hydrolysées selon l'invention sont de préférence issues de *Saccharomyces,* avantageusement de *Saccharomyces cerevisiae.*

Une composition cosmétique ou thérapeutique préférée selon l'invention comporte, à titre de substance active, des protéines de levure hydrolysées issues de levures du même genre, et de préférence du même genre et de la même espèce de levure.

Dans un autre mode de réalisation, la composition cosmétique ou thérapeutique selon l'invention comporte, à titre de substance active, des protéines de levures hydrolysées issues de levures du même genre, mais d'au moins deux espèces différentes, notamment au moins trois espèces différents.

Dans encore un autre mode de réalisation, la composition cosmétique ou thérapeutique selon l'invention comporte, à titre de substance active, des protéines de levure hydrolysées issues de levures d'au moins deux genres différents, notamment au moins trois genres différents.

La présente invention a particulièrement pour objet une composition cosmétique ou thérapeutique telle que définie ci-dessus, caractérisée en ce que les protéines de levures hydrolysées comportent au moins 40%, de préférence au moins 45%, plus préférentiellement au moins 50%, encore plus préférentiellement au moins 55%, encore plus préférentiellement au moins 60% de protéines de levure avec un poids moléculaire compris de 1 à 5 kDa.

La présente invention a particulièrement pour objet une composition cosmétique ou thérapeutique telle que définie ci-dessus, caractérisée en ce que les protéines de levures hydrolysées comportent au plus 55%, de préférence au plus 50%, plus préférentiellement au plus 45%, encore plus préférentiellement au plus 40%, encore plus préférentiellement au plus 35% de protéines de levure hydrolysées avec un poids moléculaire inférieur à 1 kDa.

La présente invention a particulièrement pour objet une composition cosmétique ou thérapeutique telle que définie ci-dessus, caractérisée en ce que le rapport AN/TN des protéines de levure hydrolysées est inférieur ou égal à 35%, notamment inférieur ou égal à 30%, notamment inférieur ou égal à 25%, notamment inférieur ou égal à 20%.

Par « rapport AN/TN », on désigne le rapport de la quantité d'azote des acides aminés (en pourcentage) sur la quantité d'azote total (en pourcentage). Le ratio AN/TN indique le taux de dégradation des protéines, en particulier le degré d'hydrolyse des protéines de levure.

Une composition préférée selon l'invention comprend des protéines de levure hydrolysées dont au moins 55% desdites protéines ont un poids moléculaire compris de 1 à 5 kDa et/ou dont au plus 42% desdites protéines ont un poids moléculaire inférieur à 1 kDa et/ou dont le rapport AN/TN est inférieur ou égal à 35%.

Une autre composition préférée selon l'invention comprend des protéines de levure hydrolysées dont au moins 60% desdites protéines ont un poids moléculaire compris de 1 à 5 kDa et/ou dont au plus 37% desdites protéines ont un poids moléculaire inférieur à 1 kDa et/ou dont le rapport AN/TN est inférieur ou égal à 35%.

Dans un mode de réalisation préféré, la présente invention a pour objet une composition cosmétique ou thérapeutique telle que définie ci-dessus, dans laquelle les protéines de levure hydrolysées sont issues du produit Springer® Hydrolyzed Yeast Peptone -A.

Le produit Springer® Hydrolyzed Yeast Peptone-A comprend des protéines de levures hydrolysées issues de la fraction insoluble de *Saccharomyces cerevisiae.* Les protéines de levure hydrolysées du produit Hydrolyzed Yeast Peptone-A comportent une majorité de protéines hydrolysées ayant un poids moléculaire supérieur ou égal à 1 kDa et inférieur à 5 kDa (environ 60%) ; les autres protéines hydrolysées ont essentiellement un poids moléculaire inférieur à lkDa (environ 32%) (voir exemple 1).

Les protéines de levure hydrolysées du produit Hydrolyzed Yeast Peptone-A sont caractérisées par un ratio AN/TN compris de 15 à 28%.

La composition selon l'invention peut comprendre le produit Springer® Hydrolyzed Yeast Peptone -A ou des protéines de levure hydrolysées obtenues par des étapes supplémentaires d'extraction et/ou purification à partir dudit produit.

La composition selon l'invention peut comprendre des protéines de levure hydrolysées correspondant à une fraction de protéines hydrolysées spécifique isolée à partir du produit Springer® Hydrolyzed Yeast Peptone -A.

Une composition cosmétique ou thérapeutique préférée selon l'invention comprend le produit Springer® Hydrolyzed Yeast Peptone -A.

La présente invention a particulièrement pour objet une composition cosmétique ou thérapeutique telle que définie ci-dessus, comprenant de 0,001% à 20% de protéines de levure hydrolysées, plus préférentiellement de 0,001 % à 15 % de protéines de levure hydrolysées, encore plus préférentiellement de 0,001% à 10% de protéines de levure hydrolysées, encore plus préférentiellement de 0,01% à 3% de protéines de levure hydrolysées, encore plus préférentiellement de 0,01% à 2% de protéines de levure hydrolysées.

Les pourcentages sont donnés en poids/poids.

La présente invention a particulièrement pour objet une composition cosmétique ou thérapeutique telle que définie ci-dessus, comprenant de 0,01 % à 20% de protéines de levure hydrolysées, notamment de 0,01 % à 15 % de protéines de levure hydrolysées, notamment de 0,01% à 10% de protéines de levure hydrolysées.

La présente invention a plus particulièrement pour objet une composition cosmétique ou thérapeutique telle que définie ci-dessus, comprenant de 0,01% à 3% de protéines de levure hydrolysées, notamment de 0,01% à 2% de protéines de levure hydrolysées, notamment de 0,01% à 1% de protéines de levure hydrolysées.

Un véhicule cosmétique ou thérapeutique acceptable selon l'invention comporte de préférence au moins un composé à titre d'additif et au moins un composé à titre d'excipient, un même composé pouvant être utilisé à plusieurs titres.

La présente invention a pour objet une composition cosmétique ou thérapeutique telle que définie ci-dessus, caractérisée en qu'elle comprend au moins un additif choisi parmi les conservateur, chélateur, colorant, filtre UV, régulateur de pH, texturant, parfum ou antioxydant, et au moins un excipient choisi parmi des composés hydrophiles, composés hydrophobes ou tensioactifs.

Par « additif », on désigne un agent ayant dans la composition cosmétique ou thérapeutique un rôle de conservateur, chélateur, colorant, filtre UV (permettant de protéger les matières premières), régulateur de pH (acide ou base), texturant, parfum et / ou antioxydant.

Par « matières premières à protéger », on désigne tout composant de la composition cosmétique ou thérapeutique susceptible d'être dégradé par la lumière.

Par « excipient », on désigne des composés hydrophiles constituant une phase aqueuse, des composés hydrophobes ou lipophiles constituant une phase grasse ou des tensioactifs.

Les tensioactifs sont des molécules amphiphiles capables de maintenir ensemble deux milieux normalement non miscibles entre eux en diminuant les tensions interfaciales.

Les tensioactifs sont ioniques (anioniques, cationiques ou amphotères) ou non-ioniques.

La liste suivante de composés pouvant être utilisés dans le véhicule cosmétique ou thérapeutique selon l'invention est donnée à titre d'exemple et ne doit pas être considérée comme exhaustive.

Les conservateurs utilisés dans les compositions selon l'invention sont notamment choisis parmi le dibutylhydroxytoluène (BHT), butylhydroxyanisole (BHA), gallates de propyle, octyle, dodécyle, α-tocophérol, acétate d'a-tocophérol, acide ascorbique, palmitate d'ascorbyle, extraits de romarin, extraits de gingko biloba, orizanol.

Les chélateurs utilisés dans les compositions selon l'invention sont notamment choisis parmi l'acide citrique, cyclodextrine, EDTA disodique, pentétate de pentasodium, acide phytique, citrate de sodium, phytate de sodium, EDTA ou pyrophosphate de tétrasodium

Les colorants utilisés dans les compositions selon l'invention sont notamment choisis parmi les colorants de dénomination CI (*Color Index*).

Les filtres UV utilisés dans les compositions selon l'invention sont notamment choisis parmi la benzophénone-3 (oxybenzone), benzophénone-4 (sulisobenzone), drométrizole, trisiloxane, salicylate de benzyle, avobenzone, octyl méthoxycinnamate (octinoxate), éthylhexyl salicylate (octisalate) ou dioxyde de titane.

Les régulateurs de pH (acide ou base) utilisés dans les compositions selon l'invention sont notamment choisis parmi l'aminomethyl propanol, acide citrique, acide fumarique, acide orthophosphorique, acide sébacique (acide décanedioïque), acétate de sodium, bicarbonate de sodium, citrate de sodium, hydroxyde de sodium, acide tartrique, pyrophosphate de tétrasodium ou triéthylamine (TEA).

Par « texturant », on désigne un agent capable d'augmenter la viscosité des phases aqueuses dans lesquelles il est dispersé, l'augmentation étant avantageusement élevée.

Un texturant peut, selon les cas, être un épaississant et/ou un gélifiant.

Par « épaississant », on désigne une substance qui permet d'obtenir une solution visqueuse sans formation d'un réseau tridimensionnel, par opposition notamment aux gélifiants.

Les texturants sont notamment choisis parmi l'agar-agar ou gélose, alginates, carraghénates, gomme de guar, gomme de tara, gomme de caroube, gomme adragante, gomme karaya, gomme de xanthane, gel d'aloès, glycérol d'amidon, chitosane, silice, silicates, - en particulier bentonite, hectorite, montmorillonite, silicate d'aluminium, silicate de magnésium -, dérivés de cellulose, notamment hydroxyéthylcellulose, hydroxypropylcellulose, méthylhydroxypropylcellulose ou hypromellose, polymères acryliques et vinyliques, - en particulier carbomères, polymères cyanoacryliques, polyvinylpyrrolidone (PVP), alcools polyvinylliques -, polyéthylène glycols, polyquatermiums.

Les parfums utilisés dans les compositions selon l'invention sont notamment choisis parmi les huiles essentielles, compositions d'origine synthétique, parfums solubilisés.

Les antioxydants utilisés dans les compositions selon l'invention sont notamment choisis parmi le palmitate d'ascorbyle, BHT, Tocophérol (Vitamine E), acétate de tocophéryl.

Les composés hydrophiles de la phase aqueuse sont notamment choisis parmi l'eau, alcools et polyols.

Les alcools pouvant être utilisés dans les compositions selon l'invention sont notamment l'éthanol, le propanol, l'isopropanol, l'alcool benzylique, l'hexyl alcool.

Les polyols pouvant être utilisés dans les compositions selon l'invention sont notamment le glycérol, le propylène glycol, le butylène glycol, l'hexylèneglycol, le sorbitol.

Les composés hydrophobes de la phase grasse sont notamment choisis parmi les hydrocarbures, acides gras, alcools gras, esters, glycérides, cérides, phosphatides.

Les hydrocarbures sont notamment choisis parmi les chaînes carbonées et hydrogénées, saturées ou insaturées, linéaires, ramifiées ou cycliques, notamment les chaînes carbonées de 22 à 35 carbone et notamment parmi les hydrocarbures suivants : paraffines, huiles de paraffine, vaselines, squalane, silicones, perhydrosqualène.

Les silicones utilisés dans les compositions selon l'invention sont notamment des huiles de silicones volatils, des huiles de silicones non volatils, des huiles de silicone modifiés, des cires de silicone, des gommes de silicone, des émulsions de silicone, des microémulsions de silicone. Les silicones sont notamment choisis parmi les silicones polyloxanes, poly diméthyl siloxanes ou diméthicones, phényl triméthyl siloxanes ou phényl méthicones, polydiméthyl siloxanes cycliques ou cyclométhicones, diméthicone copolyols, amodiméthicones, diméthicone propyl PG-Bétaïne.

Les acides gras utilisés dans les compositions selon l'invention sont notamment des acides gras saturés ou des acides gras insaturés, notamment des acides gras mono-insaturés, di-insaturés ou tri-insaturés. Les acides gras sont notamment choisis parmi les acide stéarique, acide palmitique, acide laurique, acide myristique, acide oléique, acide linoléique, acide linolénique.

Les alcools gras sont notamment choisis parmi les alccols gras à chaîne longue saturée, - alcool cétylique ou hexadécanol, alcool stéarylique, alcool cétostéarylique - , alcools gras à chaîne courte ou insaturée, - alcool oléique, octyldodécanol, alcool tétrahydrofurfurylique.

Les esters sont notamment choisis parmi les esters gras linéaires liquides, - en particulier isopropyle palmitate, myristiyl stéarate, octyl palmitate, isostéaryl isostéarate, butyl arachidonate, isopropyl lanolate, isopropyle myristate, glycéryle monostéarate - , esters de polyols, - en particulier glycérol, éthylène glycol, propylène glycol, diéthylène glycol -, les esters oxyéthylénés.

Les glycérides sont notamment des monoglycérides, diglycérides, triglycérides. Les glycérides sont notamment choisis parmi les huiles végétales, - en particulier huile d'olive, huile d'arachide, huile d'amande, huile de noisette, huile de tournesol, huile de sésame, huile de soja, huile de maïs, huile de noix, huile de pépins de raisin, huile de bourrache, huile d'onagre, huile de rosier muscat, huile de kiwi, huile d'avocat, huile germe de céréales, huile de macadamia, huile de ricin, huile oeillette, huile de coton, huile de noyau abricot, huile de coco, huile de coprah, huile de monoï, huile de palmiste, huile de carthame, huile d'andiroba, huile de pépins de courge, huile de squales, huile de vison -, les beurres, - en particulier beurre de cacao, beurre de karité, beurre de coprah, huile de babassu, huile de palme, huile de tamanu-, les huiles végétales modifiées, les huiles synthétiques, les graisses, les suifs.

Les cérides utilisés dans les compositions selon l'invention sont notamment choisis parmi les stérides, les caroténocérides, le lipochrome, les cires, - en particulier le blanc de baleine ou spermacéti, lanoline, dérivés de lanoline, - lanoline cireuse, lanoline liquide, lanoline hydrogénée, lanoline éthoxylée, alcools de lanoline, alcools de lanoline acétylés, alcools de lanoline éthoxylés, acides de lanoline, lanolate d'isopropyle -, huile de jojoba, ozokérite cérésine, cire de carnauba, cire d'abeille.

Les tensioactifs selon l'invention sont notamment des émulsionnants, mouillants, détergents et/ou moussants.

Les tensioactifs cationiques sont notamment choisis parmi les sels d'ammonium quaternaire, sels d'amines primaires grasses, amides d'ammonium quaternaire, chlorures d'alkylpyridinium, saccharinates d'alkyl ammonium, aminoxyde, amides de diéthylènetriamine ou résines cationiques.

Les tensioactifs non ioniques sont notamment choisis parmi les esters de glycérol, esters de glycols, esters de sorbitan, éthers d'alcools gras, sucroesters lipophiles, esters de polyglycérol, copolymères de l'oxyde d'éthylène-oxyde de propylène, saponines, alcools gras éthoxylés, éthers de glucose, ester de glycol ou polyéthylène glycol, ester de glycol ou polyglycérol, esters de sorbitan oxyéthylénés, alkylphénols oxyéthylénés, aminoxydes, bases autoémulsionnables (esters de PEG), dérivés du méthylglucoside, monoéthanolamides, dérivées de monoéthanolamides, diéthanolamides, ou dérivés de diéthanolamides.

Les tensioactifs anioniques sont notamment choisis parmi les savons alcalins, savons d'amines, alkylsarcosinates, alkylsulfoacétates, alkyltaurates, alkyl sulfates de sodium ou potassium, alkyl éther sulfates de sodium ou potassium, paraffine, oléfine sulfonates, iséthionates, alkyl phosphates de sodium, alkyléther phosphates de sodium.

Les tensioactifs amphotères ou zwittérioniques sont notamment choisis parmi les alkylbétaïnes, alkylamidobétaïnes, alkylamino mono ou di-propionates, dérivés imidazolés tels que cocoamphodiacétate, sodium lauroamphodiacétate.

La composition cosmétique ou thérapeutique selon l'invention peut comprendre, en plus des protéines de levure hydrolysées telles que définies ci-dessus, d'autres constituants de levure.

Dans un mode de réalisation préféré, la composition cosmétique ou thérapeutique selon l'invention ne comprend pas d'autres constituants de levure, en plus des protéines de levure hydrolysées selon l'invention.

La présente invention a particulièrement pour objet une composition cosmétique ou thérapeutique telle que définie ci-dessus, dans laquelle ladite substance active a un effet hydratant, et/ou un effet réparateur, et/ou un effet fermeté, et/ou un effet anti-vieillissement, et/ou un effet anti-séborrhée, et/ou un effet anti-acné, et/ou un effet anti-pelliculaire, et/ou un effet reconstructeur des cheveux et/ou un effet sur la brillance et/ou la douceur et/ou la croissance des cheveux.

Par l'expression « effet hydratant », on désigne une diminution de l'évaporation de la peau par un phénomène occlusif ou par une fixation de l'eau par la substance active, un effet humectant ou hygroscopique de la substance active et / ou une propriété de fixation des corps gras dans le ciment intercellulaire.

L'effet hydratant de la composition selon l'invention résulte en particulier au niveau de l'épiderme d'une activation de la synthèse lipidique, en particulier des phospholipides et des lipides neutres, et de la synthèse d'acide hyaluronique.

L'effet hydratant de la composition selon l'invention peut être mis en évidence *in vitro* par l'étude de la synthèse lipidique et d'acide hyaluronique par les kératinocytes, comme décrit dans l'Exemple 3.

L'effet hydratant de la composition selon l'invention se traduit également par un effet anti-pelliculaire lors d'une application de ladite composition sur le cuir chevelu.

L'effet anti-pelliculaire peut être mis en évidence par une diminution du nombre de pellicules chez un sujet traité avec la composition selon l'invention, par exemple comme décrit dans l'Exemple 5.

Par « effet réparateur » ou « effet cicatrisant », on désigne un effet sur la réparation et/ou la reconstruction de l'épiderme et/ou du derme. L'effet réparateur est notamment utile pour la réparation de blessures et/ou de brûlures.

L'effet réparateur des protéines de levure hydrolysées est notamment lié à l'activation de la synthèse d'acide hyaluronique. L'effet réparateur peut être mis en évidence par un dosage de la libération d'acide hyaluronique et une analyse de son expression dans des épidermes humains reconstruits, tel que décrit dans l'exemple 3.

Par l'expression « effet fermeté », on désigne un aspect lisse et tendu de la peau qui résulte de son soutien mécanique, notamment par les fibres de collagène et d'élastine.

La composition selon l'invention permet notamment d'améliorer la contraction du lattis de collagène, d'activer la synthèse d'élastine et la maturation du collagène.

Le lattis de collagène correspond à un enchevêtrement de fibres ou fibrilles de collagène.

L'effet fermeté de la composition selon l'invention peut être mis en évidence *in vitro* comme décrit dans l'exemple 3.

Par l'expression « effet anti-vieillissement » ou « effet anti-âge », on désigne à la fois un effet préventif pour retarder l'apparition des signes de vieillissement de la peau et un effet immédiat pour diminuer les signes de vieillissement. La composition selon l'invention a notamment un effet contre le vieillissement lié à l'âge et peut également avoir un effet contre le vieillissement photo-induit.

Les signes visibles du vieillissement de la peau lié à l'âge sont notamment une sécheresse cutanée, l'apparition de ridules, de rides, une diminution de l'épaisseur de la peau, ainsi qu'une perte de souplesse de la peau.

Le vieillissement de la peau lié à l'âge se traduit également par une diminution de la quantité de collagène, de leur solubilité et de leur synthèse, une diminution de la quantité d'élastine et de microfibrilles, une diminution des glycosaminoglycanes et une inactivation des fibroblastes.

L'effet anti-vieillissement de la composition selon l'invention résulte notamment d'une augmentation de la prolifération des fibroblastes du derme et de leur activité en terme de synthèse de collagène et de glycosaminoglycanes.

L'effet anti-vieillissement lié à l'âge peut être mis en évidence *in vitro* par l'augmentation de la synthèse de collagène et de glycosaminoglycanes par les fibroblastes du derme comme décrit dans l'exemple 3.

Les signes de vieillissement de la peau photo-induit sont notamment l'apparition de rides profondes, une peau épaisse et rêche.

Le vieillissement de la peau photo-induit se traduit notamment par une diminution de la quantité et de la solubilité du collagène, une augmentation de la quantité d'élastine et de microfibrilles, une augmentation des glycoaminoglycanes, une augmentation des cellules inflammatoires.

La présente invention a également pour objet une composition cosmétique ou thérapeutique telle que définie ci-dessus, caractérisée en ce que la substance active a un effet réparateur.

Par l'expression « effet anti-séborrhée », on désigne un effet de régulation de la sécrétion sébacée, de régulation de l'adsorption du sébum et / ou une action astringente permettant de resserrer les pores de la peau.

La composition selon l'invention permet de diminuer la sécrétion de sébum.

La composition selon l'invention permet notamment de réguler l'adsorption du sébum par adsorption lipidique.

La composition selon l'invention est ainsi particulièrement utile dans le cadre d'une hyperséborrhée du visage et/ou une hyperséborrhée du cuir chevelu se traduisant par des cheveux dits « gras ».

La composition cosmétique selon l'invention a un effet anti-séborrhée utile pour les peaux grasses et / ou à tendance acnéique.

Par l'expression « effet anti-acné », on désigne un effet bénéfique sur l'acné.

En particulier, l'effet bénéfique de la composition thérapeutique selon l'invention sur l'acné est lié à une régulation de la sécrétion sébacée.

Les effets anti-séborrhée et anti-acné peuvent être mis en évidence comme décrit dans l'exemple 4. Par exemple, la composition selon l'invention est appliquée sur la peau ou le cuir chevelu de sujets présentant une hyperséborrhée, respectivement au niveau de la peau ou du cuir chevelu. La sécrétion de sébum est ensuite évaluée en appliquant un patch absorbant le sébum sur la partie du corps traitée. Le patch est ensuite analysé pour quantifier la sécrétion sébacée. La sécrétion après traitement est comparée à la sécrétion chez le même sujet avant traitement.

Par « effet reconstructeur », on désigne l'obtention d'un aspect lisse des cheveux. La couche externe d'un cheveu, appelée cuticule, est composée d'écailles qui se recouvrent les unes les autres. Un effet reconstructeur se traduit par un relief lisse de la cuticule, alors que des cheveux abîmés ont un relief rugueux.

La composition selon l'invention a notamment un effet gainant sur le cheveu.

L'effet reconstructeur des cheveux peut être mis en évidence par la mesure de la topographie des cheveux, comme décrit dans l'exemple 5.

Par « effet brillance », on désigne la capacité des cheveux à réfléchir la lumière et à donner aux cheveux un effet lumineux.

Par « effet douceur », on désigne la sensation de douceur des cheveux au toucher.

Par « effet sur la croissance des cheveux », on désigne une augmentation de la cinétique de croissance des cheveux.

L'effet sur la croissance des cheveux peut être mis en évidence par un test de mesure de la cinétique de pousse des cheveux, tel que décrit dans l'exemple 5.

La présente invention a pour objet une composition cosmétique ou thérapeutique telle que définie ci-dessus sous forme de solution (une phase), dispersion (notamment une émulsion, suspension, mousse ou aérosol), gel, huile, stick, poudre, lingette, masque ou patch.

Par « émulsion », on désigne tout type d'émulsion, et notamment macro-émulsion, micro-émulsion, nano-émulsion, émulsion simple, émulsion multiple.

Les émulsions sont des dispersions d'un liquide dans un autre liquide, les deux liquides étant non miscibles. Les émulsions comportent une phase lipophile, hydrophile et un émulsionnant.

Parmi les émulsions, on trouve notamment des laits, lotions, crèmes, etc..

Les nano-émulsions sont des dispersions dont la taille des particules dispersées est inférieure à 1000 nm de diamètre, notamment de 10 nm à 100 nm.

Les micro-émulsions sont des dispersions dont la taille des particules dispersées est inférieure à 1000 µm de diamètre, notamment de 10 µm à 100 µm.

Les nano-émulsions et les micro-émulsions constituent des milieux transparents.

La composition cosmétique ou thérapeutique selon l'invention est en particulier appropriée pour des applications cutanées ou capillaires.

La composition selon l'invention pour des applications capillaires est notamment sous la forme de shampoings, lotions, masques, sprays.

La présente invention a pour objet une composition cosmétique ou thérapeutique telle que définie ci-dessus sous forme de comprimé, cachet, dragée, gélule, granule, pilule, poudre, sirops, suspension buvable, émulsion buvable.

La composition cosmétique ou thérapeutique selon l'invention peut contenir, à titre de substance active, les protéines de levure hydrolysées et au moins une substance active additionnelle.

A titre d'exemple, la ou les substances actives additionnelles peuvent avoir un effet hydratant, et/ou un effet fermeté, et/ou un effet anti-vieillissement, et/ou un effet anti-séborrhée, et/ou un effet reconstructeur des cheveux et/ou un effet sur la brillance et/ou la douceur et/ou la croissance des cheveux, et/ou réparateur, et/ou amincissant, et/ou nettoyant, et/ou anti-oxydant, et/ou dépigmentant, et/ou protecteur vasculaire, et/ou anti-inflammatoire, et/ou anti-bactérien, et/ou anti-fongique.

Dans une composition cosmétique préférée selon l'invention, au moins une substance active additionnelle a le même effet cosmétique que les protéines de levure hydrolysées.

Dans une composition thérapeutique préférée selon l'invention, au moins une substance active additionnelle a le même effet thérapeutique que les protéines de levure hydrolysées.

Lorsque les protéines de levure hydrolysées et au moins une substance active additionnelle ont le même effet cosmétique ou thérapeutique, l'effet obtenu est de préférence un effet synergique.

Les protéines de levure hydrolysées selon l'invention constituent donc un nouvel agent naturel particulièrement utile pour la préparation de compositions cosmétiques ou thérapeutiques.

La présente invention a également pour objet un procédé de préparation d'une composition cosmétique ou thérapeutique, comprenant les étapes de :
- hydrolyse de protéines de la fraction insoluble des levures, pour obtenir des protéines de levure hydrolysées, et
- mélange desdites protéines de levure hydrolysées avec un véhicule cosmétique ou thérapeutique acceptable.

Le véhicule cosmétique ou thérapeutique acceptable est en particulier choisi parmi les additifs et/ou excipients mentionnés ci-dessus.

La présente invention a également pour objet un procédé de préparation telle que défini ci-dessus d'une composition cosmétique ou thérapeutique, comprenant les étapes de :
- hydrolyse de protéines de la fraction insoluble des levures, pour obtenir des protéines de levure hydrolysées, et
- mélange desdites protéines de levure hydrolysées avec au moins une substance active additionnelle, et un véhicule cosmétique ou thérapeutique acceptable.

La présente invention a également pour objet l'utilisation de protéines de levure hydrolysées issues de la fraction insoluble des levures à titre de substance active dans des compositions cosmétiques et / ou thérapeutiques.

La présente invention a particulièrement pour objet l'utilisation telle que définie ci-dessus, caractérisée en ce que lesdites protéines de levure hydrolysées sont issues de la fraction insoluble des levures.

La présente invention a plus particulièrement pour objet l'utilisation telle que définie ci-dessus, caractérisée en ce que lesdites protéines de levure hydrolysées sont obtenues par hydrolyse enzymatique et/ou hydrolyse acide et/ou hydrolyse alcaline.

La présente invention a pour objet l'utilisation telle que définie ci-dessus, caractérisée en ce que lesdites protéines de levure hydrolysées sont obtenues par hydrolyse enzymatique avec au moins une peptidase, de préférence choisie parmi la papaïne, trypsine, chymotrypsine, subtilisine, pepsine, thermolysine, pronase, flavastacine, enterokinase, protéase facteur Xa, furine, bromélaïne, protéinase K, genenase I, thermitase, carboxypeptidase A, carboxypeptidase B, collagénase, alcalase®, neutrase® et/ou leur mélange.

La présente invention a notamment pour objet l'utilisation telle que définie ci-dessus, caractérisée en ce que lesdites protéines de levure hydrolysées sont issues de levures du genre *Saccharomyces, Kluyveromyces, Torula, Candida, Hansenula, Pichia*, et/ou leur mélange, de préférence *Saccharomyces,* avantageusement *Saccharomyces cerevisiae.*

La présente invention a également pour objet l'utilisation telle que définie ci-dessus, caractérisée en ce que lesdites protéines de levures hydrolysées comportent au moins 40%, de préférence au moins 45%, plus préférentiellement au moins 50%, encore plus préférentiellement au moins 55%, encore plus préférentiellement au moins 60% de protéines de levure avec un poids moléculaire compris de 1 à 5 kDa.

La présente invention a également pour objet l'utilisation telle que définie ci-dessus, caractérisée en ce que lesdites protéines de levures hydrolysées comportent au plus 55%, de préférence au plus 50%, plus préférentiellement au plus 45%, encore plus préférentiellement au plus 40%, encore plus préférentiellement au plus 35% de protéines de levure hydrolysées avec un poids moléculaire inférieur à 1 kDa.

La présente invention a également pour objet l'utilisation telle que définie ci-dessus, caractérisée en ce que le rapport AN/TN desdites protéines de levure hydrolysées est inférieur ou égal à 35%, notamment inférieur ou égal à 30%, notamment inférieur ou égal à 25%, notamment inférieur ou égal à 20%.

La présente invention a particulièrement pour objet l'utilisation telle que définie ci-dessus, caractérisées en ce que lesdites protéines de levure hydrolysées sont présentes dans la composition cosmétique ou thérapeutique à raison de 0,001% à 20% de, de préférence de 0,001 % à 15 % de protéines de levure hydrolysées, encore plus préférentiellement de 0,001% à 10% de protéines de levure hydrolysées, encore plus préférentiellement de 0,01% à 3% de protéines de levure hydrolysées, encore plus préférentiellement de 0,01% à 2% de protéines de levure hydrolysées.

La présente a pour objet l'utilisation telle que définie ci-dessus, caractérisée en que ladite composition cosmétique ou thérapeutique comprend au moins un additif choisi parmi les conservateur, chélateur, colorant, filtre UV, régulateur de pH, texturant, parfum ou antioxydant, et au moins un excipient choisi parmi des composés hydrophiles, des composés hydrophobes ou des tensioactifs.

La présente invention a pour objet une méthode de traitement cosmétique comprenant une étape de mise en contact sur la peau et/ou les phanères et/ou les muqueuses d'une composition cosmétique telle que définie ci-dessus ou telle que susceptible d'être obtenue par le procédé de préparation défini ci-dessus.

Le terme « mise en contact » est également appelé « application » par la suite.

La méthode de traitement peut comprendre une à plusieurs applications par jour, de préférence une à trois applications par jour.

La fréquence des applications de la composition cosmétique peut diminuer au cours du traitement.

La méthode de traitement cosmétique peut consister en un traitement court, de une à plusieurs semaines, ou un traitement à long terme sur plusieurs années. La méthode de traitement peut également consister en un traitement sous forme de cures renouvelées tous les ans ou plusieurs fois par an.

La présente invention a particulièrement pour objet une méthode de traitement cosmétique telle que définie ci-dessus, destinée à hydrater la peau et /ou les muqueuses et/ou les phanères, et/ou améliorer la réparation de la peau et/ou des muqueuses et/ou phanères, et/ou améliorer la fermeté du derme, et/ou lutter contre le vieillissement de la peau, et/ou réguler la sécrétion de sébum, et/ou diminuer les pellicules, et/ou réparer les cheveux et / ou améliorer la croissance des cheveux.

L'hydratation de l'épiderme vise à la fois à rétablir la qualité de la barrière cutanée, à savoir une imperméabilité limitant l'évaporation de l'eau, et à favoriser la présence de molécules piégeant l'eau, notamment les glycosaminoglycanes, dont l'acide hyaluronique.

La méthode de traitement cosmétique selon l'invention est particulièrement utile dans le traitement et/ou la prévention de la sécheresse cutanée et des pellicules.

La réparation de la peau et/ou des muqueuses et/ou phanères vise à aider la cicatrisation physiologique, en particulier en activant la synthèse d'acide hyaluronique.

L'affermissement du derme vise à maintenir ou renforcer la fermeté du derme, en particulier en activant la synthèse d'élastine, la synthèse et la maturation du collagène et la contraction du lattis de collagène.

La lutte contre le vieillissement de la peau a pour objet de retarder et/ou diminuer les signes de vieillissement.

Dans un mode de réalisation avantageux de l'invention, le traitement destiné à lutter contre le vieillissement est couplé à une hydratation de l'épiderme.

La méthode de traitement cosmétique selon l'invention est particulièrement préconisée chez des sujets à partir de 20 ans, notamment à partir de 30 ans, notamment à partir de 40 ans, notamment à partir de 50 ans.

La régulation de la sécrétion de sébum a notamment pour objet de diminuer la sécrétion de sébum.

La méthode de traitement cosmétique selon l'invention est particulièrement utile pour réguler la séborrhée des peaux grasses, notamment pour des peaux grasses dites « à problèmes » ou « à tendance acnéique », et/ou pour des cheveux dits « gras ».

La réparation des cheveux consiste à reconstruire le cheveu, notamment en lissant la cuticule du cheveu et /ou à redonner de la brillance et /ou de la douceur aux cheveux.

La méthode de traitement cosmétique selon l'invention est notamment appropriée chez des sujets dont les cheveux sont abîmés, notamment suite à une exposition au soleil, la mer, à des lavages trop fréquents, des colorations, des balayages, des permanentes, etc.

L'amélioration de la croissance des cheveux vise à augmenter la cinétique de croissance des cheveux, appelée également pousse des cheveux.

La méthode de traitement cosmétique selon l'invention est notamment appropriée chez des sujets dont la cinétique de croissance des cheveux est lente et/ou en cas de perte de cheveux normale.

La perte de cheveux dite normale correspond notamment à l'alopécie androgénogénétique, l'alopécie endocrinienne, ou l'alopécie liée à l'âge.

Dans un mode de réalisation avantageux, la méthode de traitement cosmétique selon l'invention est appropriée pour une application sur le visage, en particulier sur le contour des yeux, le nez, le front, le menton, sur le corps, en particulier sur les mains, les pieds, et le dos, sur les cheveux et/ou le cuir chevelu.

La présente invention a également pour objet des protéines de levure hydrolysées issues de la fraction insoluble des levures pour leur utilisation en tant que médicament, de préférence pour le traitement et/ou à la prévention de la sécheresse cutanée pathologique, des problèmes de cicatrisation pathologique et/ou l'hyperséborrhée pathologique, et/ou l'acné.

La présente invention a notamment pour objet des protéines de levure hydrolysées telles que définies ci-dessus ou telles susceptibles d'être obtenues par le procédé de préparation ci-dessus, pour le traitement et / ou la prévention de la sécheresse cutanée pathologique, des problèmes de cicatrisation pathologique et/ou l'hyperséborrhée pathologique, et/ou l'acné, et / ou de la perte des cheveux pathologique. L'invention vise encore leur utilisation pour la préparation d'une composition thérapeutique telle que définie ci-dessus.

La composition thérapeutique selon l'invention est particulièrement utile dans le traitement de la sécheresse cutanée pathologique de la peau, appelée également xérose, notamment en cas d'ichtyose, de sécheresse de la peau associée à de l'eczéma ou au psoriasis ou de sécheresse pathologique du cuir chevelu, notamment associée à des pellicules.

La composition thérapeutique selon l'invention est particulièrement utile dans le traitement de la cicatrisation pathologique, telle que la cicatrisation hypertrophique, la cicatrisation chéloïde, la cicatrisation rétractile et /ou les retards à la cicatrisation, nomment les retards liés à une mauvaise asepsie, une origine vasculaire et/ou une origine neurologique.

La composition thérapeutique selon l'invention est également utile dans le traitement de l'hyperséborrhée pathologique, notamment associé à une dérégulation hormonale, notamment chez l'adolescent, la femme enceinte ou ménopausée.

La composition thérapeutique selon l'invention est également utile dans le traitement de l'acné pathologique, notamment de l'acné juvénile ou associé à une hyperséborrhée.

La composition thérapeutique selon l'invention est également utile dans le traitement de la perte pathologique des cheveux, appelée également pelade, résultant d'un choc émotif, un dysfonctionnement thyroïdien et/ou de traitements ayant comme effet secondaire d'entraîner une alopécie (par exemple les traitement anticancéreux).

L'utilisation telle que définie ci-dessus est notamment destinée à une application topique de ladite composition thérapeutique sur la peau et/ou les phanères et/ou les muqueuses.

L'utilisation telle que définie ci-dessus peut consister en une ou plusieurs applications par jour, de préférence une à trois applications par jour.

La fréquence des applications de la composition thérapeutique peut diminuer au cours du traitement.

Le traitement thérapeutique peut consister en un traitement aigu, de quelques jours à plusieurs semaines, ou un traitement chronique sur plusieurs années. Le traitement peut également consister en un traitement sous forme de cures renouvelées tous les ans ou plusieurs fois par an.

La présente invention a également pour objet l'utilisation d'une composition cosmétique ou d'une composition thérapeutique telles que définies ci-dessus, destinée au traitement des effets secondaires ou aux manifestations désagréables d'autres traitements.

En particulier, lesdits effets secondaires ou manifestations désagréables se traduisent par une sécheresse de la peau, par exemple associée à de l'eczéma.

### EXEMPLES :

Les exemples suivants illustrent l'invention sans la limiter.

### Exemple 1 : Obtention des protéines de levure hydrolysées selon l'invention

### Matériel et Méthodes

Une suspension aqueuse de cellules de levure de *Saccharomyces cerevisiæ*, ayant une teneur en matière sèche entre 12 et 30% en masse, est soumise à un traitement thermique de 1 à 3 h entre 70°C et 90°C (afin d'inactiver les enzymes endogènes des cellules). Ce traitement thermique induit une plasmolyse de la levure, ce qui permet de séparer par la suite la fraction insoluble de la fraction soluble, la fraction soluble étant éliminée. La séparation de la fraction solubilisée de la fraction insoluble est effectuée par plusieurs étapes successives de centrifugation et lavage avec de l'eau (au moins 2 étapes successives, de préférence au moins 3).

La fraction insoluble récupérée ayant une teneur en matières sèches entre 12 et 25% en masse est ensuite hydrolysée par ajout d'au moins une protéase exogène, durant au moins 18 h à une température de 45°C à 65°C. Par exemple, la protéase est la papaïne utilisée à une concentration de 0,01% à 0,5% (poids / poids).

On sépare la fraction solubilisée hydrolysée de la fraction insoluble hydrolysée par plusieurs étapes successives de centrifugation et de lavage avec de l'eau (au moins 2 étapes successives, de préférence au moins 3).

La fraction solubilisée hydrolysée est concentrée par au moins une étape d'évaporation en continu ou en batch sous vide, pour obtenir une fraction concentrée. La fraction concentrée est éventuellement épurée par filtration ou clarification, avant d'être séchée par atomisation.

La fraction solubilisée hydrolysée et éventuellement concentrée et/ou épurée et/ou séchée ainsi obtenue correspond à des protéines de levure hydrolysées selon l'invention.

Le poids moléculaire et le profil de poids moléculaire des protéines de levure hydrolysées sont déterminés par chromatographie liquide de perméation de gel avec détection UV à 215 nm sur colonne de gel filtration Pharmacia SEPHADEX HR10/30. L'étalonnage est effectué par des standards protéiques de tailles connues, ce qui permet de calibrer le système et d'estimer le poids moléculaire d'un mélange.

Le ratio AN/TN est calculé en mesurant l'azote total et l'azote aminé.

L'azote total (TN) est déterminé par la méthode Kjeldahl, méthode établie à partir des « méthodes officielles d'analyses des produits diététiques » (JO du 3/11/79).

L'azote aminé (AN) est déterminé par dérivatisation au NQS (1-2 naphtoquinone 4-sulfonate (H.NEHRING,A. HOCK, improved method for détermination aminonitrogen, Pharmazie, 1971,26, 616-619).

### Résultats

Les protéines de levure hydrolysée selon l'invention, obtenues à partir de la fraction solubilisée hydrolysée concentrée, épurée et séchée sont désignées par la lettre « A » par la suite. Elles ont une couleur beige clair.

Le tableau 1 et la figure 1 indiquent la répartition des poids moléculaires au sein des protéines de levure hydrolysées selon l'invention (A), en comparaison avec celle de protéines de levure hydrolysées (B) issues de l'hydrolyse de la cellule entière de levure.

Les protéines de levure hydrolysées B sont obtenues par traitement thermique d'une suspension de cellules de levure *Saccharomyces cerevisiæ* de 1 à 3 h entre 70°C et 90°C, puis ajout d'au moins une protéase exogène durant au moins 18 h à une température de 45°C à 65°C. Par exemple, la protéase est la papaïne utilisée à une concentration de 0,01% à 0,5% (poids / poids). On sépare la fraction solubilisée hydrolysée de la fraction insoluble hydrolysée par plusieurs étapes successives de centrifugation et de lavage avec de l'eau (au moins 2 étapes successives, de préférence au moins 3). La fraction solubilisée hydrolysée est concentrée par au moins une étape d'évaporation en continu ou en batch sous vide, pour obtenir une fraction concentrée. La fraction concentrée est épurée par filtration ou clarification, avant d'être séchée par atomisation, pour obtenir les protéines de levure hydrolysées de levures entières (B).

Les protéines de levure hydrolysées selon l'invention (A) comportent une majorité de protéines hydrolysées ayant un poids moléculaire supérieur ou égal à 1 kDa et inférieur à 5 kDa (64,2%) ; les autres protéines hydrolysées ont essentiellement un poids moléculaire inférieur à 1kDa (31,6%).

S'agissant des protéines de levures obtenues à partir de cellules entières (B), la répartition des poids moléculaires est complètement différente : la majorité de protéines hydrolysées a un poids moléculaire inférieur à lkDa (67,3%), les autres protéines hydrolysées ayant essentiellement un poids moléculaire supérieur ou égale à 1 kDa.

**Tableau 1**

| **Poids moléculaire (en kDa)** | **Répartition (en pourcentage)** | |
|---|---|---|
| | **A** | **B** |
| ≥10 | 0,3 | 1,1 |
| ≥5 et <10 | 3,9 | 2,0 |
| ≥1 à <5 | 64,2 | 29,6 |
| <1 | 31,6 | 67,3 |

La différence entre le profil de poids moléculaire des protéines de levure hydrolysées selon l'invention et celui de protéines de levure hydrolysées issues de l'hydrolyse de cellules entières est également nettement visible sur la figure 2.

Sur la figure 2, les produits qui sont élués en premier ont les poids moléculaires les plus élevés. Les protéines de levure hydrolysées paraissent plus centrées sur une gamme de poids moléculaires élevés avec une intensité plus importante. Les protéines hydrolysées de la composition B présente une concentration de pics vers les poids moléculaires les plus faibles, ce qui est représentatif d'une plus forte dégradation.

S'agissant du rapport AN/TN, le tableau 2 indique que les protéines de levure hydrolysées selon l'invention (A) ont un ratio AN/TN compris de 15 à 28%, alors que celui des protéines de levure hydrolysées issues de cellules entière (B) est compris de 32 à 40%.

**Tableau 2**

| | **A** | **B** |
|---|---|---|
| **AN/TN** (en pourcentage) | 15-28 | 32-40 |

Le ratio AN/TN donne une estimation de la dégradation des protéines : plus il est faible, plus les protéines sont sous forme native et inversement, plus il est élevé, plus les protéines sont sous forme dégradée.

Le tableau 3 montre que les protéines de levure hydrolysées selon l'invention (A) comportent en effet très peu d'acides aminés libres, en comparaison aux protéines de levure hydrolysées issues de cellules entières (B).

Les protéines de levure hydrolysées selon l'invention (A) présentent donc un taux de dégradation moins élevé que les protéines de levure hydrolysées de la composition B.

Par ailleurs, le tableau 3 montre également que la composition en acides aminés des protéines de levure hydrolysées selon l'invention (A) est différente de celle des protéines de levure hydrolysées issues de cellules entières (B).

**Tableau 3**

| | **A (% g/g)** | | **B (% g/g)** | |
|---|---|---|---|---|
| | acides aminés libres | acides aminés totaux | acides aminés libres | acides aminés totaux |
| **ASP** | Nd | 7,59 | 0,7 | 6 |
| **SER** | Nd | 3,62 | 1,4 | 2,8 |
| **GLU** | 0,47 | 10,51 | 5,5 | 13,2 |
| **GLY** | Nd | 3 | 0,6 | 3 |
| **HIS** | Nd | 1,61 | 0,5 | 1,3 |
| **ARG** | Nd | 2,38 | 1,2 | 3,4 |
| **THR** | Nd | 4,26 | 1,1 | 3,2 |
| **ALA** | Nd | 4,75 | 2,9 | 5,1 |
| **PRO** | Nd | 2,93 | 0,5 | 3,8 |
| **CYS** | Nd | 0,2 | 0,1 | 0,3 |
| **TYR** | Nd | 1,89 | 0,3 | 1,5 |
| **VAL** | Nd | 4 | 1,3 | 3,6 |
| **MET** | Nd | 0,56 | 0,4 | 0,8 |
| **LYS** | Nd | 6,23 | 1,5 | 5 |
| **ILE** | Nd | 3,42 | 1 | 3 |
| **LEU** | Nd | 5,52 | 2,1 | 4,6 |
| **PHE** | Nd | 2,94 | 1,1 | 2,5 |
| **Total** | **0.47** | **65,41** | **22** | **63,1** |

| | | | | |
|---|---|---|---|---|
| *Nd : Non déterminé (valeurs trop faibles)* | | | | |

### Exemple 2 : Effet des protéines de levure hydrolysées selon l'invention sur le profil d'expression de kératinocytes et fibroblastes

### Matériel et méthodes

L'effet de protéines de levure hydrolysées selon l'invention sur le profil d'expression de kératinocytes épidermiques humains normaux et de fibroblastes dermiques humains normaux est évalué sur des micro-réseaux à ADN.

Le premier micro-réseau comprend 164 gènes de kératinocytes humains, notamment impliqués dans la croissance, différenciation, adhésion, communication et mort cellulaire.

Le deuxième micro-réseau comprend 143 gènes de fibroblastes humains, notamment impliqués dans la croissance, adhésion, communication, synthèse et dégradation de la matrice extracellulaire et stress.

Des kératinocytes épidermiques humains normaux et des fibroblastes dermiques humains normaux sont mis en culture pendant 24h ou 96h en présence ou en l'absence de protéines de levure hydrolysées de l'exemple 1. Les cellules sont alors lavées et leur ARN est extrait et purifié. De l'ADNc est obtenu à partir de ces ARN par transcription inverse. Les ADNc obtenus sont ensuite marqués avant d'être hybridés sur le micro-réseau correspondant au même type cellulaire.

Le niveau d'expression de chaque gène en l'absence de protéines de levure hydrolysées est comparé au niveau d'expression obtenu en présence desdites protéines de levure hydrolysées.

### Résultats

Parmi les gènes activés sur le micro-réseau des fibroblastes dermiques figurent des gènes impliqués dans la prolifération cellulaire et dans la synthèse de la matrice extracellulaire.

Les résultats obtenus sur le micro-réseau des kératinocytes épidermiques montrent que les protéines de levure hydrolysées selon l'invention stimulent la différenciation des kératinocytes épidermiques et inhibent l'expression de gènes codant pour des protéines de la matrice extracellulaire, ce qui sous-entend un effet hydratant. Le phénomène de différenciation des kératinocytes est en effet impliqué dans le renforcement de la barrière cutanée et permet de limiter les pertes en eau. L'inhibition de l'expression de gènes codant pour des protéines de la matrice extracellulaire va dans le même sens.

### Exemple 3 : Propriétés hydratantes, anti-âge et fermeté des protéines de levure hydrolysées selon l'invention

### Matériel et Méthodes

Les protéines de levure hydrolysées utilisées sont celles décrites dans l'exemple 1.

### (i) Effet hydratant

Les tests sont effectués sur des kératinocytes épidermiques humains normaux NHEK ensemencés dans les puits d'une microplaque de 96 puits dans du milieu KSFM (sans sérum). La synthèse lipidique, la synthèse de FLG (filaggrine), CK10 (cytokératine10) et TGK (transglutaminase K), et la synthèse d'acide hyaluronique sont évaluées en présence des différentes concentrations de protéines de levure hydrolysées (de 0,04 mg/ml à 1 mg/ml). Trois puits de culture sont réalisés par condition.

Du calcium est utilisé comme témoin positif de la synthèse lipidique et de la synthèse de FLG, CK10 et TGK, et l'acide rétinoïque comme témoin positif de la synthèse d'acide hyaluronique.

Le témoin négatif est constitué par du milieu de culture seul.

La synthèse lipidique est analysée par imagerie au phosphore (phosphorImaging) et la synthèse d'acide hyaluronique est évaluée par une mesure de la concentration d'acide hyaluronique libérée dans le milieu.

La synthèse de FLG et CK10 est évaluée par immunomarquage des cellules après 72h de culture, et la synthèse de TGK par immunomarquage des cellules après 48h.

### (ii) Effet anti-âge

Des fibroblastes dermiques humains normaux (NHDF) et des fibroblastes dermiques humains normaux âgés (AgNHDF) sont ensemencés dans les puits d'une microplaque de 96 puits dans du milieu DMEM + 10% de SVF. Les tests sont réalisés en milieu DMEM + 1% de SVF.

Le test de prolifération des fibroblastes et le test de synthèse de glycosaminoglycane et de collagène sont effectués en présence de différentes concentrations de protéines de levure hydrolysées. Trois puits de culture sont réalisés par condition.

Le témoin négatif est constitué par du milieu de culture seul.

Le test de prolifération est effectué 24h après ensemencement des cellules. De la [³H]-thymidine est ajoutée dans le milieu de culture. L'EGF est utilisé comme témoin positif.

La synthèse de glycosaminoglycane et de collagène est évaluée sur des cellules à 80% de confluence auxquelles est ajoutée respectivement de la [³H]-glucosamine ou de la de la [³H]-proline. L'acide rétinoïque est alors utilisé comme témoin positif.

Après 24h d'incubation, les macromolécules sont extraites et l'incorporation des précurseurs radioactifs est mesurée.

### (iii) Effet fermeté

Les tests sont effectués sur des fibroblastes dermiques humains normaux âgés (AgNHDF).

La synthèse et la maturation du collagène sont évaluées après préculture des cellules en flasque pendant 8 jours en présence de différentes concentrations de protéines de levure hydrolysées. Le témoin négatif est constitué par du milieu de culture seul et le témoin positif par du TGFβ et de la vitamine C. Les cellules sont alors ensemencées en chambre de culture. Juste avant confluence, les cellules sont fixées au méthanol et la présence de collagène est détectée par immunohistochimie en utilisant un anticorps spécifique dirigé contre le collagène I et un anticorps secondaire fluorescent. Le niveau d'expression du collagène intracellulaire et extracellulaire et sa localisation autour de la matrice sont analysés au microscope.

La contraction du lattis de collagène est évaluée après culture des cellules en flasque pendant 8 jours en présence de différentes concentrations de protéines de levure hydrolysées. Le témoin négatif est constitué par du milieu de culture seul et le témoin positif par du TGFβ. La suspension cellulaire obtenue est alors mise en présence d'une solution de collagène I sous pH contrôlé. Après quelques heures, la solution se gélifie de façon à obtenir un derme équivalent dont le contour est clairement défini. Le diamètre et le nombre de cellules de chaque derme équivalent sont mesurés en suivant une cinétique définie.

La synthèse d'élastine est évaluée après culture des cellules en flasque pendant 8 jours en présence de différentes concentrations de protéines de levure hydrolysées. Le témoin négatif est constitué par du milieu de culture seul et le témoin positif par de la vitamine C. Les cellules sont alors ensemencées en chambres de culture. Juste avant confluence, les cellules sont fixées au méthanol et la présence d'élastine est détectée par immunohistochimie en utilisant un anticorps spécifique dirigé contre l'élastine et un anticorps secondaire fluorescent. Le niveau d'expression d'élastine est analysé au microscope.

### (iv) Effet réparateur

Les tests sont effectués sur des épidermes humains reconstruits. Les épidermes reconstruits sont mis en culture. A J5, les cultures sont traitées avec les protéines de levure hydrolysées testées à 3 concentrations en application topique.

Le contrôle négatif est constitué par une culture non traitée, le témoin positif par de l'acide rétinoïque en application topique. Les traitements sont renouvelés à J7 et les cultures sont arrêtées à J10.

La libération d'acide hyaluronique dans le milieu est dosée sur les surnageants de culture au moyen d'un test Elisa modifié spécifique. Les résultats sont exprimés en µg/ml d'acide hyaluronique libérée et en pourcentage de stimulation par rapport au contrôle non traité.

L'expression d'acide hyaluronique dans les épidermes est évaluée par immunohistologie.

### (v) Statistiques

Des comparaisons intergroupes sont effectuées par analyse de variance (ANOVA) à l'aide d'un test de comparaison multiple de Dunnett.

### Résultats

### (i) Evaluation de l'effet hydratant sur l'épiderme

En présence de la solution de protéines de levure hydrolysées, la synthèse de lipides et d'acide hyaluronique par les kératinocytes est activée par rapport au contrôle négatif.

De plus, en présence de la solution de protéines de levure hydrolysées, une stimulation de la sécrétion de FLG, CK10 et TGK est observée, avec un effet dose.

### (ii) Evaluation de l'effet anti-âge sur le derme

En présence de la solution de protéines de levure hydrolysées, on observe une activation de la prolifération des cellules et une augmentation de la synthèse des constituants majeurs de la matrice extracellulaire (par rapport au contrôle négatif).

### (iii) Evaluation de l'effet fermeté

En présence de la solution de protéines de levure hydrolysées, on observe une augmentation du niveau d'expression du collagène, ainsi qu'une maturation du collagène révélée par sa déposition autour de la matrice, par rapport au témoin négatif. L'augmentation de la densité du derme équivalent (rapport diamètre sur nombre de cellules plus faible que celui du témoin négatif) traduit une meilleure contraction du lattis de collagène. De plus, la synthèse d'élastine par les fibroblastes est activée par rapport au témoin négatif. Tous ces éléments indiquent que les protéines de levure hydrolysées améliorent les qualités biomécaniques du derme (notamment en terme d'élasticité et de compressibilité).

### (iv) Evaluation de l'effet réparateur

En présence de la solution de protéines de levure hydrolysées, on observe une augmentation de l'expression d'acide hyaluronique (par rapport au contrôle négatif).

### Exemple 4 : Propriétés anti-séborrhée et anti-acné

### Matériel et Méthodes

La solution de protéines de levure hydrolysées est appliquée sur la peau ou le cuir chevelu de sujets présentant une hyperséborrhée respectivement au niveau de la peau ou du cuir chevelu.

La sécrétion de sébum est ensuite évaluée en appliquant un patch absorbant le sébum sur la partie du corps traitée. Le patch est ensuite analysé pour quantifier la sécrétion sébacée.

La sécrétion après traitement est comparée à la sécrétion chez le même sujet avant traitement.

### Résultats

La solution de protéines de levure hydrolysées permet de diminuer la quantité de sébum sécrétée.

### Exemple 5 : Applications capillaires

### Matériel et Méthodes

### (i) Protéines de levure hydrolysées

La solution de protéines de levure hydrolysées est celle décrite dans l'exemple 1.

### (ii) Effet anti-pelliculaire

La solution de protéines de levure hydrolysées est appliquée sur le cuir chevelu de sujets souffrant de pellicules. Après traitement avec la solution de protéines de levure hydrolysées, un patch est appliqué que la zone traitée pour récupérer les pellicules du cuir chevelu.

La quantité de pellicules récupérée sur le patch est comparée avant et après traitement.

### (iii) Croissance des cheveux

La cinétique de croissance des cheveux est évaluée de la manière suivante : avant traitement, une mèche de cheveu d'un sujet est colorée depuis la racine sur 2-3 cm ; la solution de protéines de levure hydrolysées est alors appliquée sur le cuir chevelu ; la distance entre la racine est le début de la coloration est mesurée.

La cinétique de croissance après traitement d'un groupe de sujet traité est comparée à celle obtenue avec un groupe de sujets non traités.

### (iv) Brillance des cheveux

La brillance des cheveux est déterminée en mesurant la quantité et l'intensité de la lumière réfléchie à la surface du cheveu. A cet effet, des photographies des cheveux sont effectuées en polarisation croisée et en non polarisation. Les deux photographies sont alors converties en niveaux de gris et la brillance des cheveux est obtenue par soustraction de la lumière entre les deux photographies.

La brillance des cheveux après application de la solution de protéines de levure hydrolysées est comparée à celle obtenue avant traitement.

### (v) Douceur des cheveux

La douceur des cheveux est évaluée en analyse sensorielle par un jury constitué de trois personnes qualifiées pour évaluer la douceur des cheveux au toucher.

La douceur des cheveux est notée sur une échelle de 0 à 10, la note de 0 correspondant à une absence de douceur et la note de 10 à une très grande douceur.

La douceur des cheveux après application de la solution de protéines de levure hydrolysées est comparée à celle obtenue avant traitement.

### (vi) Reconstruction des cheveux

La reconstruction des cheveux est évaluée en mesurant la topographie de surface des cheveux à l'aide d'un microscope interférométrique.

Les paramètres permettant de déterminer la condition des écailles de la cuticule le long du cheveu sont les suivantes :
- ouverture des écailles,
- longueur des écailles et
- topologie de surface, à savoir la rugosité.

La surface analysée mesure 120x30 µm.

La reconstruction des cheveux après application de la solution de protéines de levure hydrolysées est comparée à l'état des cheveux avant traitement.

### Résultats

L'application capillaire de la solution de protéines de levure hydrolysées permet d'obtenir un effet anti-pelliculaire et une augmentation de la croissance des cheveux.

La solution de protéines de levure hydrolysées a également un effet réparateur du cheveu, en permettant d'améliorer la brillance, la douceur et la reconstruction du cheveu. En particulier, on observe une diminution du nombre d'ouvertures des écailles de la cuticule, une augmentation de la longueur des écailles et une diminution de la rugosité.

### Exemple 6 : Exemples de compositions cosmétiques et de compositions thérapeutiques selon l'invention

Les compositions suivantes constituent des exemples non limitatifs de la présente invention.

### Composition 1 : crème hydratante (huile dans l'eau)

| Ingrédients | Pourcentage (poids/poids) |
|---|---|
| Protéines de levure hydrolysées (composition A) | 1,5 |
| Triglycéride capryl et caprique | 4 |
| Huile minérale | 2 |
| Alcool stéaryl | 3 |
| Palmitate d'isopropyle | 2 |
| Glycérol stéarate | 6 |
| PEG 100 | |
| Diméthicone | 4 |
| Glycérine | 8 |
| Conservateur | 0,3 |
| Eau | 69,2 |

### Composition 2 : lotion (huile dans l'eau)

| Ingrédients | Pourcentage (poids/poids) |
|---|---|
| Protéines de levure hydrolysées (composition A) | 2,50 |
| huile de paraffine | 2,60 |
| Propylène glycol | 1,40 |
| triglycéride | 1,00 |
| PEG-75 | 1,00 |
| Coco-caprylate caprate | 1,00 |
| Glycérol stéarate | 0,60 |
| Diméthicone | 0,50 |
| Acide polyacrylique | 0,30 |
| Hydroxyde de sodium | 0,11 |
| parfum | 0,10 |
| EDTA | 0,03 |
| glycérine | 5,00 |
| couleur | 0,32 |
| conservateur | 1,50 |
| eau purifiée | 82,04 |

### Composition 3 : shampoing antipelliculaire

| Ingrédients | Pourcentage (poids/poids) |
|---|---|
| Protéines de levure hydrolysées (composition A) | 1,5 |
| Sulfate lauryl de sodium | 30 |
| Disodium Laureth sulfate | |
| Cocoamphodiacétate | |
| Hexylène glycol | |
| Oxyde de cocamidopropylamine | 1 |
| Extrait de cresson indien | 1 |
| Conservateur | 0,2 |
| Acide citrique | pH 6 |
| eau | qsp 100 |

### Composition 4 : Masque hydratant

| Ingrédients | Pourcentage (poids/poids) |
|---|---|
| Protéines de levure hydrolysées (composition A) | 4,00 |
| Timiron flash | 4,00 |
| Propylène glycol | 3,00 |
| glycérine | 3,00 |
| urée | 3,00 |
| Acide mucique | 0,30 |
| parfum | 0,30 |
| Gomme arabique | 0,50 |
| Gomme de xanthane | 0,10 |
| EDTA | 0,10 |
| allantoïne | 0,10 |
| Hydroxyde de sodium | 0,06 |
| Alcool polyvinylique | 10,00 |
| talc | 10,00 |
| Alcool 95% | 15,00 |
| Eau purifiée | 46,54 |

## Revendications

1. Composition cosmétique ou thérapeutique comprenant 0,001% à 20% en poids de protéines d'écorces de levure hydrolysées à titre de substance active, **caractérisée en ce que** lesdites protéines d'écorces de levure hydrolysées comprennent au moins 40% en poids de protéines d'écorces de levure ayant un poids moléculaire compris entre 1 et 5 kDa et sont obtenues, à partir d'écorces de levure, par une méthode consistant en une hydrolyse enzymatique exogène et/ou une hydrolyse acide et/ou une hydrolyse alcaline, et éventuellement une séparation des protéines d'écorces de levure hydrolysées.

2. Composition selon la revendication 1, **caractérisée en ce que** la séparation des protéines d'écorces de levure hydrolysées est effectuée par centrifugation, concentration, filtration ou traitement au charbon actif.

3. Composition selon la revendication 1 ou la revendication 2, **caractérisée en ce que** l'hydrolyse enzymatique exogène est effectuée avec au moins une peptidase, de préférence choisie parmi la papaïne, trypsine, chymotrypsine, subtilisine, pepsine, thermolysine, pronase, flavastacine, enterokinase, protéase facteur Xa, furine, bromélaïne, protéinase K, generase I, thermitase, carboxypeptidase A, carboxypeptidase B, collagénase, et leurs mélanges.

4. Composition selon l'une quelconque des revendications 1 à 3, **caractérisée en ce que** les protéines d'écorces de levure hydrolysées sont obtenues à partir d'écorces de levures du genre *Saccharomyces,* du genre *Kluyveromyces,* du genre *Torula*, du genre *Candida,* du genre *Hansenula*, du genre *Pichia,* ou de leurs mélanges, de préférence à partir d'écorces de levures du genre *Saccharomyces,* avantageusement à partir d'écorces de levures *Saccharomyces cerevisiae.*

5. Composition selon l'une quelconque des revendications 1 à 4, **caractérisée en ce que** les protéines d'écorces de levures hydrolysées comprennent au moins 45% en poids, plus préférentiellement au moins 50% en poids, encore plus préférentiellement au moins 55% en poids, encore plus préférentiellement au moins 60% en poids de protéines d'écorces de levure avec un poids moléculaire compris de 1 à 5 kDa.

6. Composition selon l'une quelconque des revendications 1 à 5, **caractérisée en ce que** les protéines d'écorces de levures hydrolysées comprennent au plus 55% en poids, de préférence au plus 50% en poids, plus préférentiellement au plus 45% en poids, encore plus préférentiellement au plus 40% en poids, encore plus préférentiellement au plus 35% en poids de protéines d'écorces de levure hydrolysées avec un poids moléculaire inférieur à 1 kDa.

7. Composition selon l'une quelconque des revendications 1 à 6, **caractérisée en ce que** le rapport AN/TN des protéines d'écorces de levure hydrolysées est inférieur ou égal à 35%, notamment inférieur ou égal à 30%, notamment inférieur ou égal à 25%, notamment inférieur ou égal à 20%.

8. Composition selon l'une quelconque des revendications 1 à 7, comprenant 0,001% à 15% en poids de protéines d'écorces de levure hydrolysées, plus préférentiellement de 0,001% à 10% en poids de protéines d'écorces de levure hydrolysées, encore plus préférentiellement de 0,01% à 3% en poids de protéines d'écorces de levure hydrolysées, encore plus préférentiellement de 0,01% à 2% en poids de protéines d'écorces de levure hydrolysées.

9. Composition selon l'une quelconque des revendications 1 à 8, **caractérisée en ce qu'**elle comprend au moins un additif choisi parmi les conservateurs, chélateurs, colorants, filtres UV, régulateurs de pH, texturants, parfums et antioxydants, et/ou au moins un excipient choisi parmi des composés hydrophiles, des composés hydrophobes et des tensioactifs.

10. Procédé de préparation d'une composition cosmétique ou thérapeutique, comprenant les étapes de :
- obtention de protéines de levure hydrolysées à partir d'écorces de levure, et
- mélange desdites protéines de levure hydrolysées avec un véhicule cosmétique ou thérapeutique acceptable,
où les protéines de levure hydrolysées sont obtenues à partir d'écorces de levure par une méthode consistant en une hydrolyse enzymatique exogène et/ou une hydrolyse acide et/ou une hydrolyse alcaline, et éventuellement une séparation des protéines de levure hydrolysées.

11. Procédé selon la revendication 10, **caractérisé en ce que** la séparation des protéines de levure hydrolysées est effectuée par centrifugation, concentration, filtration ou traitement au charbon actif.

12. Procédé selon la revendication 10 ou la revendication 11, **caractérisé en ce que** l'hydrolyse enzymatique exogène est effectuée avec au moins une peptidase, de préférence choisie parmi la papaïne, trypsine, chymotrypsine, subtilisine, pepsine, thermolysine, pronase, flavastacine, enterokinase, protéase facteur Xa, furine, bromélaïne, protéinase K, generase I, thermitase, carboxypeptidase A, carboxypeptidase B, collagénase, et leurs mélanges.

13. Procédé selon l'une quelconque des revendications 10 à 12, **caractérisé en ce que** les protéines de levure hydrolysées comprennent au moins 40% en poids de protéines de levure ayant un poids moléculaire compris entre 1 et 5 kDa.

14. Utilisation de protéines de levure hydrolysées à titre de substance active dans des compositions cosmétiques, **caractérisée en ce que** les protéines de levure hydrolysées sont obtenues, à partir d'écorces de levure, par une méthode consistant en une hydrolyse enzymatique exogène et/ou une hydrolyse acide et/ou une hydrolyse alcaline, et éventuellement une séparation des protéines de levure hydrolysées.

15. Utilisation selon la revendication 14, **caractérisée en ce que** les protéines de levure hydrolysées comprennent au moins 40% en poids de protéines de levure ayant un poids moléculaire compris entre 1 et 5 kDa.

16. Méthode de traitement cosmétique non-thérapeutique comprenant une étape de mise en contact sur la peau et/ou les phanères et/ou les muqueuses d'une composition selon l'une des revendications 1 à 9 ou d'une composition obtenue par le procédé selon l'une quelconque des revendications 10 à 13.

17. Méthode de traitement cosmétique non-thérapeutique selon le revendication 16, destinée à hydrater la peau et/ou les muqueuses et/ou les phanères, et/ou des muqueuses et/ou des phanères, et/ou améliorer la fermeté du derme, et/ou lutter contre le vieillissement de la peau, et/ou réguler la sécrétion de sébum.

18. Protéines de levure hydrolysées obtenues à partir d'écorces de levure destinées à être utilisées en tant que médicament, de préférence pour le traitement et/ou la prévention de la sécheresse cutanée pathologique, pour diminuer les pellicules, des problèmes de cicatrisation pathologique et/ou l'hyperséborrhée pathologique, et/ou l'acné et/ou pour améliorer la réparation de la peau, où les protéines de levure hydrolysées sont obtenues, à partir d'écorces de levure, par une méthode consistant en une hydrolyse enzymatique exogène et/ou une hydrolyse acide et/ou une hydrolyse alcaline, et éventuellement une séparation des protéines de levure hydrolysées.

19. Les protéines de levure hydrolysées pour leur utilisation selon la revendication 18, **caractérisées en ce que** les protéines de levure hydrolysées comprennent au moins 40% en poids de protéines de levure ayant un poids moléculaire compris entre 1 et 5 kDa.

## Patentansprüche

1. Kosmetische oder therapeutische Zusammensetzung, welche 0,001 bis 20 Gewichtsprozent hydrolysierte Proteine von Hefezellwänden als wirksame Substanz enthält, **dadurch gekennzeichnet, dass** die hydrolysierten Proteine von Hefezellwänden wenigstens 40 Gewichtsprozent Proteine von Hefezellwänden mit einem Molekulargewicht zwischen 1 und 5 kDa aufweisen und aus Hefezellwänden durch ein Verfahren gewonnen werden, welches in einer exogenen enzymatischen Hydrolyse und/oder einer sauren Hydrolyse und/oder einer alkalinen Hydrolyse sowie schließlich einer Trennung der hydrolysierten Proteine von Hefezellwänden besteht.

2. Zusammensetzung nach Anspruch 1, **dadurch gekennzeichnet, dass** die Trennung der hydrolysierte Proteine von Hefezellwänden durch Zentrifugieren, Konzentrieren, Filtrieren oder Behandlung mit Aktivkohle erfolgt.

3. Zusammensetzung nach Anspruch 1 oder Anspruch 2, **dadurch gekennzeichnet, dass** die exogene enzymatische Hydrolyse mit wenigstens einer Peptidase durchgeführt wird, welche bevorzugt gewählt ist aus Papain, Trypsin, Chymotrypsin, Subtilisin, Pepsin, Thermolysin, Pronase, Flavastacin, Enterokinase, Protease-Faktor Xa, Furin, Bromelain, Proteinase K, Generase I, Thermitase, Carboxypeptidase A, Carboxypeptidase B, Kollagenase und deren Mischungen.

4. Zusammensetzung nach einem der Ansprüche 1 bis 3, **dadurch gekennzeichnet, dass** die hydrolysierten Proteine von Hefezellwänden aus Hefezellwänden der Gattung *Saccharomyces,* der Gattung *Kluyveromyces,* der Gattung *Torula*, der Gatting *Candida,* der Gattung *Hansenula,* der Gattung *Pichia* oder aus deren Mischungen gewonnen werden, bevorzugt aus Hefezellwänden der Gattung *Saccharomyces,* vorteilhafterweise aus Hefezellwänden der Gattung *Saccharomyces cerevisiae.*

5. Zusammensetzung nach einem der Ansprüche 1 bis 4, **dadurch gekennzeichnet, dass** die hydrolysierten Proteine von Hefezellwänden wenigstens 45 Gewichtsprozent, stärker bevorzugt wenigstens 50 Gewichtsprozent, noch stärker bevorzugt wenigstens 55 Gewichtsprozent, noch stärker bevorzugt wenigstens 60 Gewichtsprozent hydrolysierte Proteine von Hefezellwänden mit einem Molekulargewicht zwischen 1 und 5 kDa umfassen.

6. Zusammensetzung nach einem der Ansprüche 1 bis 5, **dadurch gekennzeichnet, dass** die hydrolysierten Proteine von Hefezellwänden höchstens 55 Gewichtsprozent, bevorzugt höchstens 50 Gewichtsprozent, stärker bevorzugt höchstens 45 Gewichtsprozent, noch stärker bevorzugt höchstens 40 Gewichtsprozent, noch stärker bevorzugt höchstens 35 Gewichtsprozent hydrolysierte Proteine von Hefezellwänden mit einem Molekulargewicht unter 1 kDa umfassen.

7. Zusammensetzung nach einem der Ansprüche 1 bis 6, **dadurch gekennzeichnet, dass** das Verhältnis AN/TN der hydrolysierten Proteine von Hefezellwänden kleiner oder gleich 35%, insbesondere kleiner oder gleich 30%, insbesondere kleiner oder gleich 25%, insbesondere kleiner oder gleich 20% ist.

8. Zusammensetzung nach einem der Ansprüche 1 bis 7, welche 0,001 bis 15 Gewichtsprozent hydrolysierte Proteine von Hefezellwänden, stärker bevorzugt 0,001 bis 10 Gewichtsprozent hydrolysierte Proteine von Hefezellwänden, noch stärker bevorzugt 0,01 bis 3 Gewichtsprozent hydrolysierte Proteine von Hefezellwänden, noch stärker bevorzugt 0,01 bis 2 Gewichtsprozent hydrolysierte Proteine von Hefezellwänden aufweist.

9. Zusammensetzung nach einem der Ansprüche 1 bis 8, **dadurch gekennzeichnet, dass** diese wenigstens einen Zusatz umfasst, welcher gewählt ist aus Konservierungsmitteln, Chelatbildnern, Farbstoffen, UV-Filtern, Stoffen zur Regulierung des pH-Wertes, Texturmitteln, Parfümen und Antioxidantien und/oder wenigstens einem Hilfsstoff, gewählt aus hydrophilen Verbindungen, hydrophoben Verbindungen und Tensiden.

10. Zubereitungsverfahren für eine kosmetische oder therapeutische Zusammensetzung, welches die folgenden Schritte umfasst:
- Erhalten von hydrolysierten Hefeproteinen aus Hefezellwänden, und
- Mischung der hydrolysierten Hefeproteine mit einem akzeptablen kosmetischen oder therapeutischen Träger,
wobei die hydrolysierten Hefeproteine aus Hefezellwänden durch ein Verfahren erhalten werden, welches in einer exogenen enzymatischen Hydrolyse und/oder einer sauren Hydrolyse und/oder einer alkalinen Hydrolyse sowie schließlich einer Trennung der hydrolysierten Hefeproteine besteht.

11. Verfahren nach Anspruch 10, **dadurch gekennzeichnet, dass** die Trennung der hydrolysierten Hefeproteine durch Zentrifugieren, Konzentrieren, Filtrieren oder Behandlung mit Aktivkohle erfolgt.

12. Verfahren nach Anspruch 10 oder Anspruch 11, **dadurch gekennzeichnet, dass** die exogene enzymatische Hydrolyse mit wenigstens einer Peptidase durchgeführt wird, welche bevorzugt gewählt ist aus Papain, Trypsin, Chymotrypsin, Subtilisin, Pepsin, Thermolysin, Pronase, Flavastacin, Enterokinase, Protease-Faktor Xa, Furin, Bromelain, Proteinase K, Generase I, Thermitase, Carboxypeptidase A, Carboxypeptidase B, Kollagenase und deren Mischungen.

13. Verfahren nach einem der Ansprüche 10 bis 12, **dadurch gekennzeichnet, dass** die hydrolysierten Hefeproteine wenigstens 40 Gewichtsprozent hydrolysierte Hefeproteine mit einem Molekulargewicht zwischen 1 und 5 kDa umfassen.

14. Verwendung von hydrolysierten Hefeproteinen als Wirkstoff in kosmetischen Zusammensetzungen, **dadurch gekennzeichnet, dass** die hydrolysierten Hefeproteine aus Hefezellwänden durch ein Verfahren erhalten werden, welches in einer exogenen enzymatischen Hydrolyse und/oder einer sauren Hydrolyse und/oder einer alkalinen Hydrolyse sowie schließlich einer Trennung der hydrolysierten Hefeproteine besteht.

15. Verwendung nach Anspruch 14, **dadurch gekennzeichnet, dass** die hydrolysierten Hefeproteine wenigstens 40 Gewichtsprozent hydrolysierte Hefeproteine mit einem Molekulargewicht zwischen 1 und 5 kDa aufweisen.

16. Kosmetische, nicht therapeutische Behandlungsmethode, welche einen Schritt des In-Kontakt-Bringens mit der Haut und/oder den Hautanhangsgebilden und/oder den Schleimhäuten einer Zusammensetzung nach einem der Ansprüche 1 bis 9 oder einer mittels des Verfahrens nach einem der Ansprüche 10 bis 13 erhaltenen Zusammensetzung umfasst.

17. Kosmetische, nicht therapeutische Behandlungsmethode nach Anspruch 16, welche dazu dient, die Haut und/oder die Schleimhäute und/oder die Hautanhangsgebilde und/oder Schleimhäute und/oder Hautanhangsgebilde zu befeuchten und/oder die Festigkeit der Dermis zu verbessern und/oder gegen die Alterung der Haut zu kämpfen und/oder die Talgdrüsensekretion zu regulieren.

18. Aus Hefezellwänden gewonnene hydrolysierte Hefeproteine, zur Verwendung als Medikament, bevorzugt für die Behandlung und/oder die Prävention von pathologischer Trockenheit der Haut, zur Verringerung von Schuppen, zur Behandlung pathologischer Narbenbildung,
und/oder pathologischer Hypersebborrhö und/oder Akne und/oder zur Verbesserung der Reparatur der Haut, wobei die hydrolysierten Hefeproteine aus Hefezellwänden durch ein Verfahren erhalten werden, welches in einer exogenen enzymatischen Hydrolyse und/oder einer sauren Hydrolyse und/oder einer alkalinen Hydrolyse sowie schließlich einer Trennung der hydrolysierten Hefeproteine besteht.

19. Hydrolysierte Hefeproteine zur Verwendung nach Anspruch 18, **dadurch gekennzeichnet, dass** die hydrolysierten Hefeproteine wenigstens 40 Gewichtsprozent, hydrolysierte Hefeproteine mit einem Molekulargewicht zwischen 1 und 5 kDa aufweisen.

## Claims

1. Cosmetic or therapeutic composition comprising 0.001% to 20% by weight of hydrolysed yeast cell wall proteins as active substance, **characterized in that** said hydrolysed yeast cell wall proteins comprise at least 40% by weight of yeast cell wall proteins having a molecular weight of between 1 and 5 kDa and are obtained from yeast cell walls by means of a method which consists of an exogenous enzymatic hydrolysis and/or an acid hydrolysis and/or an alkaline hydrolysis, and optionally a separation of the hydrolysed yeast cell wall proteins.

2. Composition according to Claim 1, **characterized in that** the separation of the hydrolysed yeast cell wall proteins is carried out by centrifugation, concentration, filtration or treatment with active carbon.

3. Composition according to Claim 1 or Claim 2, **characterized in that** the exogenous enzymatic hydrolysis is carried out with at least one peptidase, preferably chosen from papain, trypsin, chymotrypsin, subtilisin, pepsin, thermolysin, pronase, flavastacin, enterokinase, factor Xa protease, furin, bromelain, proteinase K, generase I, thermitase, carboxypeptidase A, carboxypeptidase B, collagenase, and mixtures thereof.

4. Composition according to any one of Claims 1 to 3, **characterized in that** the hydrolysed yeast cell wall proteins are obtained from cell walls of yeasts of the *Saccharomyces* genus, *Kluyveromyces* genus, *Torula* genus, *Candida* genus, *Hansenula* genus, *Pichia* genus, or mixtures thereof, preferably from cell walls of yeasts of the *Saccharomyces* genus, advantageously from *Saccharomyces cerevisiae* yeast cell walls.

5. Composition according to any one of Claims 1 to 4, **characterized in that** the hydrolysed yeast cell wall proteins comprise at least 45% by weight, more preferentially at least 50% by weight, even more preferentially at least 55% by weight, even more preferentially at least 60% by weight of yeast cell wall proteins with a molecular weight of from 1 to 5 kDa.

6. Composition according to any one of Claims 1 to 5, **characterized in that** the hydrolysed yeast cell wall proteins comprise at most 55% by weight, preferably at most 50% by weight, more preferentially at most 45% by weight, even more preferentially at most 40% by weight, even more preferentially at most 35% by weight of hydrolysed yeast cell wall proteins with a molecular weight of less than 1 kDa.

7. Composition according to any one of Claims 1 to 6, **characterized in that** the AN/TN ratio of the hydrolysed yeast cell wall proteins is less than or equal to 35%, in particular less than or equal to 30%, in particular less than or equal to 25%, in particular less than or equal to 20%.

8. Composition according to any one of Claims 1 to 7, comprising 0.001% to 15% by weight of hydrolysed yeast cell wall proteins, more preferentially from 0.001% to 10% by weight of hydrolysed yeast cell wall proteins, even more preferentially from 0.01% to 3% by weight of hydrolysed yeast cell wall proteins, even more preferentially from 0.01% to 2% by weight of hydrolysed yeast cell wall proteins.

9. Composition according to any one of Claims 1 to 8, **characterized in that** it comprises at least one additive chosen from preservatives, chelating agents, dyes, UV-screening agents, pH regulators, texturing agents, fragrances and antioxidants, and/or at least one excipient chosen from hydrophilic compounds, hydrophobic compounds and surfactants.

10. Process for preparing a cosmetic or therapeutic composition, comprising the steps of:
- obtaining hydrolysed yeast proteins from yeast cell walls, and
- mixing said hydrolysed yeast proteins with an acceptable cosmetic or therapeutic carrier,
wherein the hydrolysed yeast proteins are obtained from yeast cell walls by means of a method which consists of an exogenous enzymatic hydrolysis and/or an acid hydrolysis and/or an alkaline hydrolysis, and optionally a separation of the hydrolysed yeast proteins.

11. Process according to Claim 10, **characterized in that** the separation of the hydrolysed yeast proteins is carried out by centrifugation, concentration, filtration or treatment with active carbon.

12. Process according to Claim 10 or Claim 11, **characterized in that** the exogenous enzymatic hydrolysis is carried out with at least one peptidase, preferably chosen from papain, trypsin, chymotrypsin, subtilisin, pepsin, thermolysin, pronase, flavastacin, enterokinase, factor Xa protease, furin, bromelain, proteinase K, generase I, thermitase, carboxypeptidase A, carboxypeptidase B, collagenase, and mixtures thereof.

13. Process according to any one of Claims 10 to 12, **characterized in that** the hydrolysed yeast proteins comprise at least 40% by weight of yeast proteins having a molecular weight of between 1 and 5 kDa.

14. Use of hydrolysed yeast proteins as active substance in cosmetic compositions, **characterized in that** the hydrolysed yeast proteins are obtained from yeast cell walls by means of a method which consists of an exogenous enzymatic hydrolysis and/or an acid hydrolysis and/or an alkaline hydrolysis, and optionally a separation of the hydrolysed yeast proteins.

15. Use according to Claim 14, **characterized in that** the hydrolysed yeast proteins comprise at least 40% by weight of yeast proteins having a molecular weight of between 1 and 5 kDa.

16. Non-therapeutic cosmetic treatment method comprising a step of bringing into contact with the skin and/or the skin appendages and/or the mucous membranes a composition according to one of Claims 1 to 9 or a composition obtained by means of the process according to any one of Claims 10 to 13.

17. Non-therapeutic cosmetic treatment method according to Claim 16, intended for moisturising the skin and/or the mucous membranes and/or the skin appendages, and/or mucous membranes and/or skin appendages, and/or improving the firmness of the skin, and/or combating skin ageing, and/or regulating sebum secretion.

18. Hydrolysed yeast proteins obtained from yeast cell walls, intended to be used as a medicament, preferably for the treatment and/or prevention of pathological dryness of the skin, for decreasing dandruff, pathological healing problems and/or pathological hyperseborrhoea, and/or acne and/or for improving skin repair, wherein the hydrolysed yeast proteins are obtained from yeast cell walls by means of a method which consist of an exogenous enzymatic hydrolysis and/or an acid hydrolysis and/or an alkaline hydrolysis, and optionally a separation of the hydrolysed yeast proteins.

19. Hydrolysed yeast proteins for use thereof according to Claim 18, **characterized in that** the hydrolysed yeast proteins comprise at least 40% by weight of yeast proteins having a molecular weight of between 1 and 5 kDa.
